# FASCICULE DE BREVET EUROPEEN

(11) **EP 2 714 930 B1**
(45) Date de publication et mention de la délivrance du brevet: **01.02.2017**
(21) Numéro de dépôt: 12729671.3
(22) Date de dépôt: 25.05.2012
(51) Int. Cl.: C12Q 1/68

(54) **AMORCES UNIVERSELLES ET LEUR UTILISATION POUR LA DÉTECTION ET L'IDENTIFICATION D'ESPÈCES DE VERTÉBRÉS**
UNIVERSELLE PRIMER UND IHRE VERWENDUNG FÜR DEN NACHWEIS UND DIE IDENTIFIZIERUNG VON WIRBELTIERARTEN
UNIVERSAL PRIMERS AND THE USE THEREOF FOR THE DETECTION AND IDENTIFICATION OF VERTEBRATE SPECIES

(30) Priorité: 26.05.2011 FR 1154606
(43) Date de publication de la demande: 09.04.2014
(73) Titulaire: Universite Joseph Fourier (Grenoble 1), 38041 Grenoble (FR); Centre National de la Recherche Scientifique, 75016 Paris (FR)
(72) Inventeur: TABERLET, Pierre, F-38660 La Terrasse (FR); COISSAC, Eric, F-73110 Arvillard (FR); RIAZ, Tiayyba, F-38000 Grenoble (FR)
(74) Mandataire: Agasse, Stéphane
(86) Numéro de dépôt international: PCT/FR2012/000212
(87) Numéro de publication internationale: WO 2012/160277

(56) Documents cités:
- EP-A2- 2 113 573
- GB-A- 2 374 597
- US-A1- 2003 143 534
- TAKASHI KITANO ET AL: "Two universal primer sets for species identification among vertebrates", INTERNATIONAL JOURNAL OF LEGAL MEDICINE, SPRINGER, BERLIN, DE, vol. 121, no. 5, 15 juillet 2006 (2006-07-15), pages 423-427, XP019541912, ISSN: 1437-1596
- PEREIRA F ET AL: "Analysis of inter-specific mitochondrial DNA diversity for accurate species identification", INTERNATIONAL CONGRESS SERIES, EXCERPTA MEDICA, AMSTERDAM, NL, vol. 1288, 1 avril 2006 (2006-04-01), pages 103-105, XP025082698, ISSN: 0531-5131, DOI: 10.1016/J.ICS.2005.09.125 [extrait le 2006-04-01]
- MITANI T ET AL: "Identification of animal species using the partial sequences in the mitochondrial 16S rRNA gene", LEGAL MEDICNE, JAPANESE SOCIETY OF LEGAL MEDICINE, TOKYO, JP, vol. 11, 1 avril 2009 (2009-04-01), pages S449-S450, XP026134658, ISSN: 1344-6223, DOI: 10.1016/J.LEGALMED.2009.02.002 [extrait le 2009-03-16]
- RODRIGUEZ M A ET AL: "TaqMan real-time PCR for the detection and quantitation of pork in meat mixtures", MEAT SCIENCE, ELSEVIER SCIENCE, GB, vol. 70, no. 1, 1 mai 2005 (2005-05-01), pages 113-120, XP025283296, ISSN: 0309-1740, DOI: 10.1016/J.MEATSCI.2004.12.005 [extrait le 2005-05-01]
- LOPEZ-CALLEJA ET AL: "Quantitative detection of goats' milk in sheep's milk by real-time PCR", FOOD CONTROL, BUTTERWORTH, LONDON, GB, vol. 18, no. 11, 5 mai 2007 (2007-05-05), pages 1466-1473, XP022060160, ISSN: 0956-7135, DOI: 10.1016/J.FOODCONT.2006.11.006
- MARTIN ET AL: "Mitochondrial markers for the detection of four duck species and the specific identification of Muscovy duck in meat mixtures using the polymerase chain reaction", MEAT SCIENCE, ELSEVIER SCIENCE, GB, vol. 76, no. 4, 27 avril 2007 (2007-04-27) , pages 721-729, XP022050651, ISSN: 0309-1740, DOI: 10.1016/J.MEATSCI.2007.02.013
- LOPEZ-CALLEJA ET AL: "Real-time TaqMan PCR for quantitative detection of cows' milk in ewes' milk mixtures", INTERNATIONAL DAIRY JOURNAL, ELSEVIER APPLIED SCIENCE, BARKING, GB, vol. 17, no. 7, 31 mars 2007 (2007-03-31), pages 729-736, XP022011491, ISSN: 0958-6946, DOI: 10.1016/J.IDAIRYJ.2006.09.005
- BERRY N ET AL: "Mitochondrial DNA and retroviral RNA analyses of archival oral polio vaccine (OPV CHAT) materials: evidence of macaque nuclear sequences confirms substrate identity", VACCINE, ELSEVIER LTD, GB, vol. 23, no. 14, 25 février 2005 (2005-02-25), pages 1639-1648, XP004739795, ISSN: 0264-410X, DOI: 10.1016/J.VACCINE.2004.10.038

### SEQUENCE LISTING

<110> Université Joseph Fourier CNRS
<120> Amorces universelles et leur utilisation pour la détection et l'identification d'espèces de vertébrés
<130> BR075259/NJO/CCU
<160> 142
<170> PatentIn version 3.5
<210> 1
   <211> 18
   <212> DNA
   <213> Artificial Sequence
<220>
   <223> primer
<400> 1
   tagaacaggc tcctctag 18
<210> 2
   <211> 18
   <212> DNA
   <213> Artificial sequence
<220>
   <223> primer
<400> 2
   ttagataccc cactatgc 18
<210> 3
   <211> 18
   <212> DNA
   <213> Artificial Sequence
<220>
   <223> sequence complementary to a primer
<400> 3
   ctagaggagc ctgttcta 18
<210> 4
   <211> 18
   <212> DNA
   <213> Artificial Sequence
<220>
   <223> sequence complementary to a primer
<400> 4
   gcatagtggg gtatctaa 18
<210> 5
   <211> 18
   <212> DNA
   <213> Artificial Sequence
<220>
   <223> primer
<400> 5
   ttagataccc tactatgc 18
<210> 6
   <211> 18
   <212> DNA
   <213> Artificial Sequence
<220>
   <223> primer
<400> 6
   taggacaggc tcctctag 18
<210> 7
   <211> 18
   <212> DNA
   <213> Artificial Sequence
<220>
   <223> primer
<400> 7
   caggacaggc tcctctag 18
<210> 8
   <211> 18
   <212> DNA
   <213> Artificial Sequence
<220>
   <223> primer
<400> 8
   cagaacaggc tcctctag 18
<210> 9
   <211> 97
   <212> DNA
   <213> Alytes obstetricians
<400> 9
   ctagccttaa acattgacaa cttacattat ttcatccgcc cgggaactac gagccgcagc 60
   ttaaaaccca aaggacttgg cggtgcccca aacccac 97
<210> 10
   <211> 95
   <212> DNA
   <213> Bombina variegata
<400> 10
   ctaacctcaa actatgactc aatacaaaac accatccgcc cgggaactac gagccctagc 60
   ttaaaaccca aaggacttgg cggtgcccca cccac 95
<210> 11
   <211> 89
   <212> DNA
   <213> Bufo bufo
<400> 11
   ccagccgtaa acttttactt acatcaaatc gccagggaac tacgagccaa gcttaaaacc 60
   caaaggactt gacggtaccc cacatcccc 89
<210> 12
   <211> 90
   <212> DNA
   <213> Bufo viridis
<400> 12
   ccagccgtaa acttttattt acatccaaat cgcccgggaa ctacgagcta agcttaaaac 60
   ccaaaggact tgacggtacc ccatatcccc 90
<210> 13
   <211> 90
   <212> DNA
   <213> Dendrobates tinctorius
<400> 13
   tcagtcgtaa actttaaatt acttcctact cgcccgggaa ctacgagcta tgcttaaaac 60
   ccaaaggact tgacggtacc ccatatcccc 90
<210> 14
   <211> 90
   <212> DNA
   <213> Hyla arborea
<400> 14
   tcagccgtaa actttaaatt acattcgtat cgcctgggaa ctacgagcca agcttaaaac 60
   ccaaaggact tgacggtacc ccacatccac 90
<210> 15
   <211> 90
   <212> DNA
   <213> Hyla meridionalis
<400> 15
   tcagccgtaa actttaaatt acaccaatat cgccagggaa ctacgagcta agcttaaaac 60
   ccaaaggact tgacggtacc ccatatccac 90
<210> 16
   <211> 90
   <212> DNA
   <213> Rana pipiens
<400> 16
   ctagccgtaa acaattaatt tacacccatc agcgccaggg aactacgagc aatgcttaaa 60
   acccaaagga tttgacggtg tcccacccag 90
<210> 17
   <211> 89
   <212> DNA
   <213> Rana temporaria
<400> 17
   ctagccgtaa acaattaact cacatccaca ccgccaggga attacgagca atgcttaaaa 60
   cccaaaggat ttgacggtgt cccacccca 89
<210> 18
   <211> 83
   <212> DNA
   <213> Salamandra atra
<400> 18
   ctagacataa actttgatta tccgccagag tactacgagc tacagcttaa aactcaaagg 60
   acttggcggt gctttacacc ccc 83
<210> 19
   <211> 83
   <212> DNA
   <213> Salamandra lanzai
<400> 19
   ctaaacataa actttgatta tccgccagag tactacgagc cacagcttaa aactcaaagg 60
   acttggcggt gctttacacc ccc 83
<210> 20
   <211> 101
   <212> DNA
   <213> Accipiter gentilis
<400> 20
   ctggccctaa atcttgatac ttactttaca aaagtatccg cccgagaact acgagcacaa 60
   acgcttaaaa ctctaaggac ttggcggtgc cccaaaccca c 101
<210> 21
   <211> 102
   <212> DNA
   <213> Acrocephalus arundinaceus
<400> 21
   ctggccctaa atcttgatgc tcaaccttac cgaagcatcc gcccgagaac tacgagcgca 60
   aacgcttaaa actctaagga cttggcggtg tcccaaaccc ac 102
<210> 22
   <211> 104
   <212> DNA
   <213> Alectoris rufa
<400> 22
   ctggccttaa atcttgatac ccccccattt accaatatat ccgcccgaga actacgagca 60
   caaacgctta aaactctaag gacttggcgg tgccccaaac ccac 104
<210> 23
   <211> 101
   <212> DNA
   <213> Anser anser
<400> 23
   ctggccctaa atcttgatac ttactttacc gaagtatccg ccagagaact acgagcacaa 60
   acgcttaaaa ctctaaggac ttggcggtgc cccaaaccca c 101
<210> 24
   <211> 101
   <212> DNA
   <213> Aquila chrysaetos
<400> 24
   ctggccctaa atcttaatac ttcccccact aaagtattcg cccgagtact acgagcacaa 60
   acgcttaaaa ctctaaggac ttggcggtgc cccataccca c 101
<210> 25
   <211> 101
   <212> DNA
   <213> Ardea cinerea
<400> 25
   tcagccttaa atcttggtac ttaacttact aaagtatccg cctgagaact acgagcacaa 60
   acgcttaaaa ctctaaggac ttggcggtgc cccaaaccca c 101
<210> 26
   <211> 101
   <212> DNA
   <213> Asio flammeus
<400> 26
   caggccctaa atcctaatgc tcccaccacc agagcattcg ccagggcact acgagcacaa 60
   acgcttaaaa ccctaaggac ttggcggtac cctaaaccca c 101
<210> 27
   <211> 101
   <212> DNA
   <213> Asio otus
<400> 27
   caggccctaa atcccaatgc tcctttcacc agagcattcg ccagggtact acgagcacaa 60
   acgcttaaaa ccctaaggac ttggcggtac cccaaaccca c 101
<210> 28
   <211> 102
   <212> DNA
   <213> Bubo bubo
<400> 28
   ccggccctaa atccagatgc ccacagtcac taaagcatcc gccagggtac tacgagcaca 60
   aacgcttaaa accctaagga cttggcggtg ccccaaaccc ac 102
<210> 29
   <211> 101
   <212> DNA
   <213> Buteo buteo
<400> 29
   ctggccctaa atcctgatac ttatcttacc aaagtatccg cccgagaact acgagcacaa 60
   acgcttaaaa ctctaaggac ttggcggtgc cccaaaccca c 101
<210> 30
   <211> 102
   <212> DNA
   <213> cardinalis cardinalis
<400> 30
   ctggccctaa atcttgatgc ttacacctac taaagcatcc gcccgagaac tacgagcact 60
   aacgcttaaa actctaagga cttggcggtg ctccaaaccc ac 102
<210> 31
   <211> 101
   <212> DNA
   <213> Ciconia ciconia
<400> 31
   ttagccctaa atcttggtac ttaccctact aaagtacccg cccgagaact acgagcacaa 60
   acgcttaaaa ctctaaggac ttggcggtgc cccaaaccca c 101
<210> 32
   <211> 101
   <212> DNA
   <213> Columba livia
<400> 32
   ctggccctaa atctagatgc ttatgttact aaagcatccg cccgaggact acgagcacaa 60
   acgcttaaaa ctctaaggac ttggcggtgc cctaaaccca c 101
<210> 33
   <211> 102
   <212> DNA
   <213> Corvus corone
<400> 33
   ctggccctaa atcttgatgc tctatgctac ctgagcatcc gcccgagaac tacgagcaca 60
   aacgcttaaa actctaagga cttggcggtg tcccaaaccc ac 102
<210> 34
   <211> 101
   <212> DNA
   <213> Cygnus cygnus
<400> 34
   ctggccctaa atcttgatac ttactttacc gaagtatccg ccagagaact acgagcacaa 60
   acgcttaaaa ctctaaggac ttggcggtgc cccaaaccca c 101
<210> 35
   <211> 103
   <212> DNA
   <213> Falco peregrinus
<400> 35
   ctagccctaa atcctgatgt ttattgtacc atagcacatc cgcccgagaa ttacgagcac 60
   aaacgcttga aactctaagg acttggcggt gccccaaacc cac 103
<210> 36
   <211> 102
   <212> DNA
   <213> Fringilla coelebs
<400> 36
   ctggccctaa atcttgatgc ttacccctac taaagcatcc gcccgagaac tacgagcacc 60
   aacgcttaaa actctaagga cctggcggtg ccccaaaccc ac 102
<210> 37
   <211> 102
   <212> DNA
   <213> Gallus gallus
<400> 37
   ctagccctaa atctagatac ctcccatcac acatgtatcc gcctgagaac tacgagcaca 60
   aacgcttaaa actctaagga cttggcggtg ccccaaaccc ac 102
<210> 38
   <211> 100
   <212> DNA
   <213> Lagopus muta
<400> 38
   ctggccctta atccagatac ccatataccc ctgtatccgc ccgagaacta cgagcacaaa 60
   cgcttaaaac tctaaggact tggcggtgcc ctaaacccac 100
<210> 39
   <211> 102
   <212> DNA
   <213> Motacilla alba
<400> 39
   ctggccctaa atcttgatgc ttacccctac taaagcatcc gcccgagaac tacgagcacc 60
   aacgcttaaa actctaagga cttggcggtg ccccaaaccc ac 102
<210> 40
   <211> 102
   <212> DNA
   <213> Parus major
<400> 40
   ctggccctaa atcttgatgc tcgatattac ctgagcatcc gcctgagaac tacgagcaca 60
   aacgcttaaa actctaagga cttggcggtg ccccaaaccc ac 102
<210> 41
   <211> 102
   <212> DNA
   <213> Passer domesticus
<400> 41
   ctggccctaa atcttgatgc ttaaacctac taaagcatcc gcccgagaac tacgagcact 60
   aacgcttaaa actctaagga cttggcggtg ccccaaaccc ac 102
<210> 42
   <211> 100
   <212> DNA
   <213> Perdix perdix
<400> 42
   ctggccctaa atctagacac ccgcacaccc gagtgtccgc ctgagaacta cgagcacaaa 60
   cgcttaaaac tctaaggact tggcggtgcc ctaaacccac 100
<210> 43
   <211> 100
   <212> DNA
   <213> Phasianus colchicus
<400> 43
   ctggccctaa atctagatgc ctacataccc atgcatccgc ccgagaacta cgagcacaaa 60
   cgcttaaaac tctaaggact tggcggtgcc ccaaacccac 100
<210> 44
   <211> 102
   <212> DNA
   <213> Sylvia atricapilla
<400> 44
   ctggccctaa atcttgatgc tcgatcttac tggagcatcc gcccgagaac tacgagcacc 60
   aacgcttaaa actctaagga cttggcggtg ccccacatcc ac 102
<210> 45
   <211> 100
   <212> DNA
   <213> Tetrao urogallus
<400> 45
   ctggccctta atctagatac ctatataccc ttgtatccgc ctgagaacta cgagcacaaa 60
   cgcttaaaac tctaaggact tggcggtgcc ctaaacccac 100
<210> 46
   <211> 102
   <212> DNA
   <213> Turdus philomelos
<400> 46
   ctggccctaa atcttgatgc tcgatattac ctgagcatcc gcccgagaac tacgagcaca 60
   aacgcttaaa actctaagga cttggcggtg ccccaaaccc ac 102
<210> 47
   <211> 102
   <212> DNA
   <213> zosterops pallidus
<400> 47
   ctggccctaa atcttgatgc tcgatctaac cggagcatcc gcccgagaac tacgagcaca 60
   aacgcttaaa actctaagga cttggcggtg ctccaaatcc ac 102
<210> 48
   <211> 97
   <212> DNA
   <213> Chimaera monstrosa
<400> 48
   ttagccctaa acctaggtgc ttaattacct aaacacccgc cagagaacta caagcgccag 60
   cttgaaaccc aaaggacttg gcggtgctta gatcccc 97
<210> 49
   <211> 101
   <212> DNA
   <213> Manta birostris
<400> 49
   ttaaccataa acattgttat taatgatata ccttaataac cgcctgagta ctacaagcgc 60
   tagcttaaaa cccaaaggac ttggcggtgc tccaaatcca c 101
<210> 50
   <211> 105
   <212> DNA
   <213> Raja brachyura
<400> 50
   ttaaccataa atagaccttt actaccactt actttgttta agtccgcctg agtactacaa 60
   gcgctagctt aaaacccaaa ggacttggcg gtgccccaga ccccc 105
<210> 51
   <211> 98
   <212> DNA
   <213> Squatina nebulosa
<400> 51
   ccatctacaa acttagacaa taatctacca tattgtccgc cagagtacta caagcgctag 60
   cttaaaaccc aaaggacttg gcggtgcctc agaccctc 98
<210> 52
   <211> 96
   <212> DNA
   <213> Torpedo tokionis
<400> 52
   ttaattataa atagataaat attcactaac tcattcgccc gagaactaca agcgctagct 60
   taaaacccaa aggacttggc ggcacttcag atctac 96
<210> 53
   <211> 100
   <212> DNA
   <213> Bison bison
<400> 53
   ttagccctaa acacagataa ttacataaac aaaattattt gccagagtac taccagcaac 60
   agcttaaaac tcaaaggact tggcggtgct ttatatcctt 100
<210> 54
   <211> 100
   <212> DNA
   <213> Bos taurus
<400> 54
   ttagccctaa acacagataa ttacataaac aaaattattc gccagagtac tactagcaac 60
   agcttaaaac tcaaaggact tggcggtgct ttatatcctt 100
<210> 55
   <211> 99
   <212> DNA
   <213> Canis lupus
<400> 55
   ttagccctaa acataaacca ttctataaca aaataattcg ccagagaact actagcaaca 60
   gcttaaaact caaaggactt ggcggtgctt tatatccct 99
<210> 56
   <211> 100
   <212> DNA
   <213> Capra hircus
<400> 56
   ttagccctaa acacaaataa ttacagaaac aaaattattc gccagagtac taccggcaac 60
   agcccgaaac tcaaaggact tggcggtgct ttataccctt 100
<210> 57
   <211> 100
   <212> DNA
   <213> Capra ibex
<400> 57
   ttagccctaa acacaagtag tcacaaaaac aagattattc gccagagtac taccggcaac 60
   agcccgaaac tcaaaggact tggcggtgct ttataccctt 100
<210> 58
   <211> 100
   <212> DNA
   <213> Capreolus capreolus
<400> 58
   ttagccctaa acacaagtaa ttaatataac aaaattattc gccagagtac taccggcaat 60
   agcttaaaac tcaaaggact tggcggtgct ttataccctt 100
<210> 59
   <211> 100
   <212> DNA
   <213> Castor fiber
<400> 59
   ttagccgtaa acccaaatat gtaaaccaac aaacatattc gccagagaac tactagcaac 60
   agcttaaaac tcaaaggact tggcggtgct ttatatccgt 100
<210> 60
   <211> 100
   <212> DNA
   <213> Cervus elaphus
<400> 60
   ctagccttaa acacaaatag ttatgcaaac aaaactattc gccagagtac taccggcaat 60
   agcttaaaac tcaaaggact tggcggtgct ttataccctt 100
<210> 61
   <211> 98
   <212> DNA
   <213> Eliomys quercinus
<400> 61
   tcagccctaa acacaaaaaa ttcacaacaa aattgttcgc cagagaacta cgagccacag 60
   ctcaaaactc aaaggacttg gcggtgcttt atatccct 98
<210> 62
   <211> 98
   <212> DNA
   <213> Eptesicus serotinus
<400> 62
   ttagccctaa acatggaaaa ttataaacaa aattattcgc cagagtacta ccagcaagag 60
   ctagaaactc aaaggacttg gcggtgcctt aagtcctt 98
<210> 63
   <211> 98
   <212> DNA
   <213> Erinaceus europaeus
<400> 63
   ttagccctaa acttagacag ttacttaaca aaactgtacg ccagagaact acgagctaca 60
   gcttaaaact caaaggactt ggcggtgctt tatacccc 98
<210> 64
   <211> 101
   <212> DNA
   <213> Felis catus
<400> 64
   ttagccctaa acttagatag ttatcctaaa caaaactatc cgccagagaa ctactagcaa 60
   tagcttaaaa ctcaaaggac ttggcggtgc tttacatccc t 101
<210> 65
   <211> 98
   <212> DNA
   <213> Glis glis
<400> 65
   ttagccctaa acataaaccc ttactaacgc aaccgttcgc cagagtacta caagccacag 60
   ctcaaaactc aaaggacttg gcggtgcttt atatccct 98
<210> 66
   <211> 100
   <212> DNA
   <213> Homo sapiens
<400> 66
   ttagccctaa acctcaacag ttaaatcaac aaaactgctc gccagaacac tacgagccac 60
   agcttaaaac tcaaaggacc tggcggtgct tcatatccct 100
<210> 67
   <211> 99
   <212> DNA
   <213> Lepus europaeus
<400> 67
   ttagccttaa acctaaataa tttcctaaca aaattattcg ccagagaact acaagccaga 60
   gcttaaaact caaaggactt ggcggtgctt tatacccgc 99
<210> 68
   <211> 100
   <212> DNA
   <213> Lutra lutra
<400> 68
   tcagccctaa acatagatag cttacataac aaaactatct gccagagaac tactagcaac 60
   agcttaaaac tcaaaggact tggcggtgct ttacatccct 100
<210> 69
   <211> 100
   <212> DNA
   <213> Meles meles
<400> 69
   tcagccctaa acatagataa ttcatagaac aaaattatct gccagagaac tactagcaac 60
   agcttaaaac tcaaaggact tggcggtgct ttacatccct 100
<210> 70
   <211> 101
   <212> DNA
   <213> Mus musculus
<400> 70
   ttagccataa acctaaataa ttaaatttaa caaaactatt tgccagagaa ctactagcca 60
   tagcttaaaa ctcaaaggac ttggcggtac tttatatcca t 101
<210> 71
   <211> 100
   <212> DNA
   <213> Mustela erminea
<400> 71
   tcagccctaa acataaatag ttctaacaac aaaactatct gccagagaac tactagcaac 60
   agcttaaaac tcaaaggact tggcggtgct ttacatccct 100
<210> 72
   <211> 98
   <212> DNA
   <213> Myodes glareolus
<400> 72
   ttagccctaa acttcatcat tataaaacaa aagtatttgc ctgagaacta ctagccacag 60
   cttaaaactc aaaggacttg gcggtacttt atatccat 98
<210> 73
   <211> 99
   <212> DNA
   <213> Oryctolagus cuniculus
<400> 73
   ttagccctaa actttgataa tttcataaca aaattattcg ccagagaact acaagccaaa 60
   gcttaaaact caaaggactt ggcggtgctt tatacccac 99
<210> 74
   <211> 99
   <212> DNA
   <213> Ovis aries
<400> 74
   ttagccctaa acacaaataa ttataaaaac aaaattattc gccagagtac taccgcaaca 60
   gcccgaaact caaaggactt ggcggtgctt tataccctt 99
<210> 75
   <211> 100
   <212> DNA
   <213> Rattus norvegicus
<400> 75
   ttagccctaa accttaataa ttaaacctac aaaattattt gccagagaac tactagctac 60
   agcttaaaac tcaaaggact tggcggtact ttatatccat 100
<210> 76
   <211> 99
   <212> DNA
   <213> Sciurus vulgaris
<400> 76
   ttagccctaa acatagacac tcaattaaca agactgttcg ccagaggact actagctact 60
   gcttaaaact caaaggactt ggcggtgctt tacatccct 99
<210> 77
   <211> 100
   <212> DNA
   <213> Sorex araneus
<400> 77
   ttagccctaa actcaggtat ttaacctaac aaaaataccc gccagagaac tactagcaat 60
   agcttaaaac tcaaaggact tggcggtgct ttatatccct 100
<210> 78
   <211> 99
   <212> DNA
   <213> Sus scrofa
<400> 78
   ctagccctaa acccaaatag ttacataaca aaactattcg ccagagtact actcgcaact 60
   gcctaaaact caaaggactt ggcggtgctt cacatccac 99
<210> 79
   <211> 99
   <212> DNA
   <213> ursus arctos
<400> 79
   ttagccttaa acataaataa tttattaaac aaaattattc gccagagaac tactagcaac 60
   agcttaaaac tcaaaggact tggcggtgct ttaaccccc 99
<210> 80
   <211> 96
   <212> DNA
   <213> Coronella austriaca
<400> 80
   ctaaccataa cacaacaatt aaatcactta ttgttcgcca aataactacg agtaaaaact 60
   taaaatttaa aagacttgac ggtacctcaa aacaac 96
<210> 81
   <211> 96
   <212> DNA
   <213> Coronella girondica
<400> 81
   ctaaccataa cacaacaatc aaataaccca ttgttcgcca aataactacg agtaaaaact 60
   taaaatttaa aagacttgac ggtacctcaa aacaac 96
<210> 82
   <211> 102
   <212> DNA
   <213> Gekko gecko
<400> 82
   ccagacgtaa actttgatag aaccacatca caaatctatc cgccagggga ctactagtaa 60
   ctaacttaaa actccaagga cttgacggtg ttccatatca tc 102
<210> 83
   <211> 100
   <212> DNA
   <213> Lacerta schreiberi
<400> 83
   taagccataa acattgatta gccactatac aacttctccg ccagagaatt acaagtgaaa 60
   aacttaaaac tcaaaggact tgacggtgtc ccatatcgac 100
<210> 84
   <211> 96
   <212> DNA
   <213> Naja naja
<400> 84
   ctagccgtaa caaaacaatt aaataactaa ttgttcgcca aataactacg agtgaaaact 60
   taaaatttaa aagacttgac ggtgcttcac atcaac 96
<210> 85
   <211> 93
   <212> DNA
   <213> Natrix maura
<400> 85
   ctaacctaac acacaataaa ttaccaattg ttcgccaaac aactacgagt aaaaacttaa 60
   aacttaaaag acttgacggt acttcacacc acc 93
<210> 86
   <211> 93
   <212> DNA
   <213> Natrix natrix
<400> 86
   ctaacctaac acacaataaa ctaccaattg ttcgccaaac aactacgagt aaaaacttaa 60
   aacttaaaag acttgacggt acttcacacc acc 93
<210> 87
   <211> 101
   <212> DNA
   <213> Podarcis muralis
<400> 87
   taagccctga acattgatag ttactaatac aatactttcc gccagagaac tacaagtgaa 60
   aaacttaaaa ctcaaaggac ttgacggtgt cccatatcgg c 101
<210> 88
   <211> 95
   <212> DNA
   <213> vipera ursinii
<400> 88
   ccaaccacaa cccacaatta aaatactaat tgttcgccaa acaactacga gtagtaactt 60
   aaaacttaaa agacttgacg gtactttatc acaac 95
<210> 89
   <211> 99
   <212> DNA
   <213> Acipenser persicus
<400> 89
   tcagtcataa actttggtga taaattacac atattgcccg ccagggtact acgagcgcta 60
   gcttaaaacc caaaggactt ggcggtgccc cagacccac 99
<210> 90
   <211> 99
   <212> DNA
   <213> Anguilla anguilla
<400> 90
   tcaaccttaa acaacgatga caacatacaa atatcatccg ccaggggact acgagcgtta 60
   gcttaaaacc caaaggactt ggcggtgcct caaacccac 99
<210> 91
   <211> 100
   <212> DNA
   <213> Barbus barbus
<400> 91
   tcagccgtaa acttagacat ccaaatacaa ttagatgtcc gccagggtac tacgagcacc 60
   agcttaaaac ccaaaggacc tgacggtgtc tcagaccccc 100
<210> 92
   <211> 99
   <212> DNA
   <213> coregonus lavaretus
<400> 92
   ctagccgtaa actttgatgg aaacatacaa ctaacatccg ccagggaact acaagcgcca 60
   gcttaaaacc caaaggactt ggcggtgcct cagacccac 99
<210> 93
   <211> 99
   <212> DNA
   <213> Cottus gobio
<400> 93
   ctagccctaa acattgatag tacaatacac ccactatccg cccgggtact acgagcaata 60
   gcttaaaacc caaaggactt ggcggtgctt tagatccac 99
<210> 94
   <211> 99
   <212> DNA
   <213> Gadus morhua
<400> 94
   atggtcgtta acattgatgg ttttataccc aaaccgtccg cctgggaact acgagcaata 60
   gcttaaaacc caaaggactt ggcggtgctt tagaccccc 99
<210> 95
   <211> 99
   <212> DNA
   <213> Gasterosteus aculeatus
<400> 95
   ctagccctaa acattgatag aattttacaa ccactatccg cccggggact acaagcatca 60
   gcttgaaacc caaaggactt ggcggtgctt tagatccac 99
<210> 96
   <211> 99
   <212> DNA
   <213> Labrus merula
<400> 96
   ttagccgtaa acattgatag tacagtacat ctactatccg cccgggtact acgagcacca 60
   gcttaaaacc caaaggactt ggcggtgctt tagatccac 99
<210> 97
   <211> 99
   <212> DNA
   <213> Lota lota
<400> 97
   atggttgtta acattgatgg ttttatactt aaaccatccg cctgggtact acgagcatta 60
   gcttaaaacc caaaggactt ggcggtgctt tagaccccc 99
<210> 98
   <211> 99
   <212> DNA
   <213> Oncorhynchus mykiss
<400> 98
   ctagccgtaa accttgatag aaatatacaa ttgatatccg ccagggaact acaagcgcca 60
   gcttaaaacc caaaggactt ggcggtgcct cagacccac 99
<210> 99
   <211> 99
   <212> DNA
   <213> salvelinus fontinalis
<400> 99
   ctagccgtaa actttgatag aaaaatacaa ctgatatccg ccagggaact acaagcgcca 60
   gcttaaaacc caaaggactt ggcggtgcct cagacccac 99
<210> 100
   <211> 94
   <212> DNA
   <213> solea solea
<400> 100
   ttagccataa acatcgatct ttacacaaaa atccgcccgg gaactacaaa cacgagttta 60
   aaacccaaag gacttggcgg tgcttaacat ccac 94
<210> 101
   <211> 45
   <212> DNA
   <213> Artificial Sequence
<220>
   <223> primer
<400> 101
   ctatgcttag ccctaaacct caacagttaa atcaacaaaa ctgct 45
<210> 102
   <211> 17
   <212> DNA
   <213> Artificial Sequence
<220>
   <223> primer
<400> 102
   ccgcccgtca cyctcct 17
<210> 103
   <211> 21
   <212> DNA
   <213> Artificial Sequence
<220>
   <223> primer
<400> 103
   gtayrcttac cwtgttacga c 21
<210> 104
   <211> 20
   <212> DNA
   <213> Artificial Sequence
<220>
   <223> primer
<400> 104
   cgagaagacc ctatggagct 20
<210> 105
   <211> 17
   <212> DNA
   <213> Artificial Sequence
<220>
   <223> primer
<400> 105
   ccgaggtcrc cccaacc 17
<210> 106
   <211> 19
   <212> DNA
   <213> Artificial Sequence
<220>
   <223> primer
<400> 106
   acctggattr ctccggtct 19
<210> 107
   <211> 19
   <212> DNA
   <213> Artificial Sequence
<220>
   <223> primer
<400> 107
   acctcgatgt tggatcagg 19
<210> 108
   <211> 23
   <212> DNA
   <213> Artificial Sequence
<220>
   <223> primer
<400> 108
   cggtctgaac tcagatcacg tag 23
<210> 109
   <211> 22
   <212> DNA
   <213> Artificial Sequence
<220>
   <223> primer
<400> 109
   gaaaatgtag cccatttctt cc 22
<210> 110
   <211> 17
   <212> DNA
   <213> Artificial Sequence
<220>
   <223> primer
<400> 110
   cataccgccg tcgccag 17
<210> 111
   <211> 20
   <212> DNA
   <213> Artificial sequence
<220>
   <223> primer
<400> 111
   cagggtcttc tcgtcttatg 20
<210> 112
   <211> 20
   <212> DNA
   <213> Artificial Sequence
<220>
   <223> primer
<400> 112
   gtatgaatgg ctaaacgagg 20
<210> 113
   <211> 17
   <212> DNA
   <213> Artificial Sequence
<220>
   <223> primer
<400> 113
   cgcccgtcac cctcytc 17
<210> 114
   <211> 22
   <212> DNA
   <213> Artificial Sequence
<220>
   <223> primer
<400> 114
   ttaccttgtt acgacttacc tc 22
<210> 115
   <211> 22
   <212> DNA
   <213> Artificial Sequence
<220>
   <223> primer
<400> 115
   tatgataaag tgaacatrga gg 22
<210> 116
   <211> 21
   <212> DNA
   <213> Artificial Sequence
<220>
   <223> primer
<400> 116
   ggttcraytc ctrcttttct a 21
<210> 117
   <211> 22
   <212> DNA
   <213> Artificial Sequence
<220>
   <223> primer
<400> 117
   gatatctttc aggtgtaagc tg 22
<210> 118
   <211> 22
   <212> DNA
   <213> Artificial Sequence
<220>
   <223> primer
<400> 118
   aggtaagtcg taacaaggta ag 22
<210> 119
   <211> 22
   <212> DNA
   <213> Artificial Sequence
<220>
   <223> primer
<400> 119
   tacgagcaca aacgcttaaa ac 22
<210> 120
   <211> 18
   <212> DNA
   <213> Artificial Sequence
<220>
   <223> primer
<400> 120
   gacggcggta tgtaggct 18
<210> 121
   <211> 17
   <212> DNA
   <213> Artificial Sequence
<220>
   <223> primer
<400> 121
   acaccgcccg tcaccct 17
<210> 122
   <211> 23
   <212> DNA
   <213> Artificial Sequence
<220>
   <223> primer
<400> 122
   gtayacttac catgttacga ctt 23
<210> 123
   <211> 30
   <212> DNA
   <213> Artificial Sequence
<220>
   <223> primer
<400> 123
   tcaccctcct caagtatact tcaaaggaca 30
<210> 124
   <211> 20
   <212> DNA
   <213> Artificial Sequence
<220>
   <223> primer
<400> 124
   gggattagat accccactat 20
<210> 125
   <211> 21
   <212> DNA
   <213> Artificial Sequence
<220>
   <223> primer
<400> 125
   tcaagtcctt tgggttttaa g 21
<210> 126
   <211> 29
   <212> DNA
   <213> Artificial Sequence
<220>
   <223> primer
<400> 126
   ccactatgct tagccctaaa cctcaacag 29
<210> 127
   <211> 21
   <212> DNA
   <213> Artificial Sequence
<220>
   <223> primer
<400> 127
   cgagaagacc ctatggagct t 21
<210> 128
   <211> 20
   <212> DNA
   <213> Artificial Sequence
<220>
   <223> primer
<220>
   <221> misc_feature
   <222> (17)..(17)
   <223> n is a, c, g, or t
<400> 128
   cryggtcgcc ccaaccnaaa 20
<210> 129
   <211> 39
   <212> DNA
   <213> Artificial Sequence
<220>
   <223> primer
<400> 129
   tggagcttta atttattaat gcaaacagta cctaacaaa 39
<210> 130
   <211> 17
   <212> DNA
   <213> Artificial Sequence
<220>
   <223> primer
<400> 130
   acaccgcccg tcactct 17
<210> 131
   <211> 20
   <212> DNA
   <213> Artificial Sequence
<220>
   <223> primer
<400> 131
   cttccggtac acttaccatg 20
<210> 132
   <211> 18
   <212> DNA
   <213> Artificial Sequence
<220>
   <223> primer
<400> 132
   aaactcgtgc cagccacc 18
<210> 133
   <211> 18
   <212> DNA
   <213> Artificial Sequence
<220>
   <223> primer
<400> 133
   aaactcgtgc cagccacc 18
<210> 134
   <211> 20
   <212> DNA
   <213> Artificial Sequence
<220>
   <223> primer
<400> 134
   gggtatctaa tcccagtttg 20
<210> 135
   <211> 27
   <212> DNA
   <213> Artificial Sequence
<220>
   <223> primer
<400> 135
   gccaccgcgg tcacacgatt aacccaa 27
<210> 136
   <211> 27
   <212> DNA
   <213> Artificial Sequence
<220>
   <223> primer
<400> 136
   gccaccgcgg tcatacgatt aacccaa 27
<210> 137
   <211> 18
   <212> DNA
   <213> Artificial Sequence
<220>
   <223> primer
<400> 137
   acaccgcccg tcactctc 18
<210> 138
   <211> 21
   <212> DNA
   <213> Artificial Sequence
<220>
   <223> primer
<400> 138
   catgttacga cttgcctcct c 21
<210> 139
   <211> 33
   <212> DNA
   <213> Artificial Sequence
<220>
   <223> primer
<400> 139
   accctcctca agtatacttc aaaggacatt taa 33
<210> 140
   <211> 45
   <212> DNA
   <213> Artificial Sequence
<220>
   <223> primer
<400> 140
   ctatgcttag ccctaaacct agatagttag cccaaacaaa actat 45
<210> 141
   <211> 45
   <212> DNA
   <213> Artificial Sequence
<220>
   <223> primer
<400> 141
   ctatgcttag ccctaaacct agatagttag ctcaaacaaa actat 45
<210> 142
   <211> 46
   <212> DNA
   <213> Artificial Sequence
<220>
   <223> primer
<400> 142
   ctatgcttag ccctaaactt agatagttaa ttttaacaaa actatc 46

## Description

La présente invention concerne des oligonucléotides et leur utilisation comme amorces universelles pour la détection et l'identification d'espèces de vertébrés, notamment dans des substrats complexes et dégradés.

L'identification taxonomique basée sur l'analyse de l'ADN est une approche couramment utilisée aujourd'hui pour identifier des espèces au sein d'un mélange. Un consortium international nommé Barcode for Life a été créé avec comme objectif de permettre la détection d'espèces animales ou végétales à partir d'une séquence d'ADN. Pour identifier les animaux, l'approche consiste à amplifier puis à séquencer une séquence de 648 paires de base du gène mitochondrial cytochrome c oxidase 1 ("CO1") CO1 s'est montré efficace pour l'identification des oiseaux, papillons, poissons, mouches et autres groupes animaux (Hebert *et al*., 2003). Pour les végétaux, la situation est plus complexe et deux autres séquences d'ADN chloroplastiques, *mat*K and *rbc*L, ont été proposées (Hollingsworth *et al.,* 2009). Il s'avère néanmoins que les fragments choisis selon cette méthode sont de longueur trop longue (supérieure à 500 nucléotides) pour être utilisés dans des substrats dégradés. Or, dans de très nombreuses situations, les substrats à analyser contiennent de l'ADN dégradé, avec souvent des fragments de moins de 100 nucléotides. C'est le cas notamment des échantillons collectés dans l'environnement (sol, eau, fèces) ou des matrices utilisées dans l'industrie, par exemple les farines animales ou les plats industriels de l'industrie alimentaire.

Une solution pour la détection et l'identification spécifique consiste à analyser des fragments d'ADN informatifs de petite taille (Poinar *et al*., 1998; Willersley *et al.,* 2003 ; Willersley *et al*., 2007). Des amorces ont ainsi été définies afin d'amplifier des fragments d'ADN végétaux plus courts dans des échantillons de sols gelés. Néanmoins, les amorces permettent d'identifier les familles mais pas les espèces végétales. En outre, les amorces n'ont pas été choisies de manière à amplifier des séquences suffisamment conservées dans le règne végétal pour permettre l'amplification de toute espèce végétale. En d'autres mots, ces amorces ne sont pas véritablement universelles.

WO 2006/024751 décrit un procédé permettant de détecter simultanément dans un échantillon de matière biologique la présence éventuelle de matières biologiques par réaction de polymérisation en chaîne (PCR) puis hybridation avec des sondes. Les amorces décrites sont très dégénérées (pratiquement chaque codon comporte un nucléotide dégénéré) et manquent par conséquent de spécificité pour l'amplification de l'ADN de vertébrés.

Le brevet EP1797201 propose des oligonucléotides permettant la détection et l'identification de végétaux dans des substrats complexes ou dégradés car la région amplifiée est à la fois courte et très variable. Plus précisément, la région amplifiée correspond à une région variable de l'intron du gène chloroplastique *trn*L du tabac. A cet égard, référence est faite également à l'article de Taberlet *et al*., 2007.

Les amorces décrites dans ce brevet sont spécifiques des espèces végétales et elles ne permettent pas l'identification et la détection des espèces de vertébrés dans des substrats complexes ou dégradés.

Takashi Kitano et al. décrivent une méthode utilisant des olignucléotides universels pour l'identification d'espèces basée sur la PCR. La méthode décrite permet l'amplification de fragments de taille allant de 215 à 244 pb. Ces fragments sont trop longs pour permettre une identification si le substrat est dégradé.

Pereira et al. divulguent une méthode visant à l'identification d'espèces de mammifères basée sur l'utilisation de fragments d'ADN mitochondrial de type 12S d'une taille inférieure à 200 pb amplifiés à l'aide de paires d'oligonucléotide définies dans le tableau. Cependant, les paires d'oligonucléotides décrites permettent d'amplifier des fragments d'ADN mitochondrial de type 12S de vertébrés et non uniquement de mammifères.

La présente invention propose de nouveaux oligonucléotides et leurs utilisations comme amorces universelles pour l'identification et la détection des espèces vertébrés. Ces amorces permettent à la fois d'amplifier une région courte (moins de 120 paires de base), une région très variable entre les espèces vertébrées et une région présentant dans le même temps des régions flanquantes très conservées permettant l'amplification à l'aide une seule et unique paire d'amorces. Ainsi ces amorces peuvent être utilisées dans des mélanges complexes et dégradés, par exemple des échantillons de sols, de fèces et d'eau. De telles amorces trouvent notamment des applications dans l'analyse des plats industriels susceptibles de contenir des mélanges de viandes ou de poissons, mais également pour l'analyse des régimes alimentaires des carnivores à partir des fèces.

Plus précisément, ces amorces permettent d'amplifier une région 12S de l'ADN mitochondrial. Il est particulièrement intéressant de disposer d'amorces permettant l'amplification d'ADN mitochondrial car celui-ci représente une cible très accessible dans le cas de substrats dégradés. De plus, l'ADN mitochondrial est présent de manière répétée dans chaque cellule.

Les amorces universelles objet de la présente invention sont particulièrement avantageuses car elles sont extrêmement spécifiques des vertébrés et n'amplifient aucun autre groupe taxonomique.

### Description de l'invention

La présente concerne une paire d'oligonucléotides, selon laquelle le premier oligonucélotide s'hybride de manière sélective à la séquence SEQ ID NO. 3 et le deuxième nucléotide s'hybride de manière sélective à la séquence SEQ ID NO. 4 dans des conditions de stringences suffisantes pour l'amplification d'une région variable du gène mitochondrial 12S des vertébrés par réaction de polymérisation en chaîne (PCR).

Selon un aspect de l'invention, la région amplifiée du gène mitochondrial 12S comporte moins de 120 nucléotides (amorces non comprises).

Selon un autre aspect de l'invention, le premier oligonucléotide présente une séquence nucléotidique SEQ ID NO. 1 ou SEQ ID NO. 6-8 et le deuxième oligonucléotide présente une séquence nucléotidique sélectionnés dans le groupe consistant en SEQ ID NO. 2 et SEQ ID NO. 5.

Selon un autre aspect encore de l'invention, la région amplifiée par réaction de polymérisation en chaîne présente une séquence nucléotidique sélectionnée dans le groupe consistant SEQ ID NO. 9-100.

La présente invention concerne également un procédé d'amplification d'une région du gène mitochondrial 12S, comprenant les étapes suivantes :
a) on dispose d'un échantillon susceptible de contenir de l'ADN d'espèces vertébrés ;
b) on réalise une réaction d'amplification en chaîne en utilisant une paire d'oligonucléotides selon l'invention.

La présente invention propose également un procédé de détection d'une espèce vertébrée dans un échantillon, comprenant les étapes suivantes :
a) on dispose d'un échantillon susceptible de contenir de l'ADN d'espèces vertébrés ;
b) on isole l'ADN total contenu dans l'échantillon ;
c) on réalise une réaction d'amplification en chaîne en utilisant une paire d'oligonucléotides selon l'invention ; et
d) on détecte l'éventuelle présence d'un produit d'amplification.

La présente invention concerne également un procédé de détection et d'identification d'une espèce vertébrée dans un échantillon, comprenant les étapes suivantes :
a) on dispose d'un échantillon susceptible de contenir de l'ADN d'espèces vertébrés ;
b) on isole l'ADN total contenu dans l'échantillon ;
c) on réalise une réaction d'amplification en chaîne en utilisant une paire d'oligonucléotides selon l'invention;
d) on détecte la présence d'un produit d'amplification ; et
e) on détermine la séquence du produit d'amplification pour identifier l'espèce de vertébré contenue dans l'échantillon.

Un autre objet de la présente invention concerne également un kit pour la détection d'une espèce de vertébré dans un échantillon, comprenant une paire d'oligonucléotides selon l'une quelconque des revendications 1 à 4 et au moins un réactif additionnel.

Selon un aspect de l'invention, le kit comprend une amorce de blocage selon SEQ ID NO. 101.

Selon un autre aspect de l'invention, le kit comprend en outre au moins au moins une autre paire d'oligonucléotides choisis parmi les paires d'amorces :
- amorces d'amplification selon SEQ ID NO.102 et SEQ ID NO.103;
- amorces d'amplification selon SEQ ID NO.104 et SEQ ID NO.105;
- amorces d'amplification selon SEQ ID NO.106 et SEQ ID NO.107, éventuellement avec amorce de blocage selon SEQ ID NO.108 ;
- amorces d'amplification selon SEQ ID NO.109 et SEQ ID NO.110;
- amorces d'amplification selon SEQ ID NO.111 et SEQ ID NO.112;
- amorces d'amplification selon SEQ ID NO.113 et SEQ ID NO.114;
- amorces d'amplification selon SEQ ID NO.115 et SEQ ID NO.116;
- amorces d'amplification selon SEQ ID NO.117 et SEQ ID NO.118;
- amorces d'amplification selon SEQ ID NO.119 et SEQ ID NO.120;
- amorces d'amplification selon SEQ ID NO.121 et SEQ ID NO.122, éventuellement avec amorce de blocage selon SEQ ID NO.123 ;
- amorces d'amplification selon SEQ ID NO.124 et SEQ ID NO.125, éventuellement avec amorce de blocage selon SEQ ID NO.126 ;
- amorces d'amplification selon SEQ ID NO.127 et SEQ ID NO.128, éventuellement avec amorce de blocage selon SEQ ID NO.129 ;
- amorces d'amplification selon SEQ ID NO.130 et SEQ ID NO.131, éventuellement avec amorce de blocage selon SEQ ID NO.132 ;
   a) on dispose d'un échantillon susceptible de contenir de l'ADN d'espèces vertébrés ;
   b) on isole l'ADN total contenu dans l'échantillon ;
   c) on réalise une réaction d'amplification en chaîne en utilisant une paire d'oligonucléotides selon l'invention;
   d) on détecte la présence d'un produit d'amplification ; et
   e) on détermine la séquence du produit d'amplification pour identifier l'espèce de vertébré contenue dans l'échantillon.

Un autre objet de la présente invention concerne également un kit pour la détection d'une espèce de vertébré dans un échantillon, comprenant une paire d'oligonucléotides selon l'une quelconque des revendications 1 à 4 et au moins un réactif additionnel.

Selon un aspect de l'invention, le kit comprend une amorce de blocage selon SEQ ID NO. 101.

Selon un autre aspect de l'invention, le kit comprend en outre au moins au moins une autre paire d'oligonucléotides choisis parmi les paires d'amorces :
- amorces d'amplification selon SEQ ID NO.102 et SEQ ID NO.103;
- amorces d'amplification selon SEQ ID NO.104 et SEQ ID NO.105;
- amorces d'amplification selon SEQ ID NO.106 et SEQ ID NO.107, éventuellement avec amorce de blocage selon SEQ ID NO.108 ;
- amorces d'amplification selon SEQ ID NO.109 et SEQ ID NO.110;
- amorces d'amplification selon SEQ ID NO.111 et SEQ ID NO.112;
- amorces d'amplification selon SEQ ID NO.113 et SEQ ID NO.114;
- amorces d'amplification selon SEQ ID NO.115 et SEQ ID NO.116;
- amorces d'amplification selon SEQ ID NO.117 et SEQ ID NO.118;
- amorces d'amplification selon SEQ ID NO.119 et SEQ ID NO.120;
- amorces d'amplification selon SEQ ID NO.121 et SEQ ID NO.122, éventuellement avec amorce de blocage selon SEQ ID NO.123 ;
- amorces d'amplification selon SEQ ID NO.124 et SEQ ID NO.125, éventuellement avec amorce de blocage selon SEQ ID NO.126 ;
- amorces d'amplification selon SEQ ID NO.127 et SEQ ID NO.128, éventuellement avec amorce de blocage selon SEQ ID NO.129 ;
- amorces d'amplification selon SEQ ID NO.130 et SEQ ID NO.131, éventuellement avec amorce de blocage selon SEQ ID NO.132 ;
dérivés les polynucléotides de la présente invention flanquent une région à la fois courte et très variable de l'ADN des vertébrés, plus précisement une région courte et variable dans le gène 12S mitochondrial des vertébrés. La variablité de cette région entre espèces de vertébrés peut donc être utilisée pour détecter et identifier les espèces de vertébrés.

Selon la présente invention, on entend par « oligonucléotide » une chaîne nucléotidique simple brin ou son complémentaire pouvant être de type ADN ou ARN, ou une chaîne nucléotidique double brin pouvant être de type ADN complémentaire ou génomique. Selon un mode réalisation, les oligonucléotides de l'invention sont de type ADN, notamment ADN double brin. Le terme « oligonucléotide » désigne également les polynucléotides modifiés. Les oligonucléotides modifiés sont par exemple des oligonucléotides conjugués à des réactifs de liaison (biotine par exemple) ou à des réactifs marqués (marqueurs fluorescents par exemple). Classiquement, les réactifs de liaison ou les réactifs marqués conjugués aux oligonucléotides facilitent la purification ou la détection de ces oligonucléotides.

Les oligonucléotides de la présente invention peuvent être préparés par synthèse chimique ou par des techniques classiques de biologie moléculaire telles que décrites par Sambrook, Fristsch et Maniatis, dans leur ouvrage intitulé "Molecular cloning: a laboratory manual", édition : Cold Spring Harbor Laboratory Press, Cold Spring Harbor, NY, 1989. Les oligonucléotides de la présente invention peuvent également être isolés ou purifiés de leur environnement naturel.

Le terme « polynucléotide » peut ici être utilisé en remplacement du terme « oligonucléotide » avec une signification équivalente.

On entend par « amorce » une courte séquence d'oligonucléotides qui, hybridée avec une matrice d'acide nucléique, permet à une polymérase d'entamer la synthèse d'un nouveau brin de l'ADN. Le brin produit à partir de l'amorce est complémentaire au brin utilisé comme matrice. Les amorces sont notamment utilisées dans les réactions de polymérisation en chaîne (PCR).

On entend par « espèce de vertébré » ou « espèce vertébrée » tout organisme faisant partie du sous-embranchement vertébré du règne animal.

La présente invention concerne donc une paire d'oligonucléotides selon laquelle le premier oligonucléotide s'hybride de manière sélective à une région très conservée du gène mitochondrial 12S des vertébrés et le deuxième nucléotide s'hybride de manière sélective à une autre région très conservée du gène mitochondrial 12S des vertébrés dans des conditions de stringences suffisantes pour l'amplification de la région variable du gène mitochondrial 12S des vertébrés par réaction de polymérisation en chaîne (PCR), par exemple dans des conditions de forte stringence. La séquence de la première région conservée correspond à la séquence de l'amorce 12S-V05_F (SEQ ID NO. 1) et de sa séquence complémentaire (SEQ ID NO. 3). La séquence de la deuxième région conservée correspond à la séquence de l'amorce 12S-V05_R (SEQ ID NO.2) et de sa séquence complémentaire (SEQ ID NO. 4).

A titre d'exemple, chez l'Homme, le premier oligonucléotide de la présente invention se trouve à la position 1194 de la séquence NC_012920, Homo sapiens mitochondrion et le second oligonucléotide de l'invention se trouve à la position 1093 de cette même séquence.

L'homme du métier connaît les réactions d'amplification d'ADN et les conditions de stringence permettant une amplification sélective d'une séquence. L'homme du métier connait en particulier les conditions de température d'hybridation et les conditions de composition du tampon d'hybridation.

L'homme du métier pourra donc facilement définir différentes variantes des amorces 12S-V05_F (SEQ ID NO. 1) et 12S-V05_R (SEQ ID NO. 2) en utilisant des techniques de routine. Ces variantes s'hybrident aux séquences de référence et permettent l'amplification sélective de la région variable d'intérêt de l'ADN mitochondrial 12S.

Une variante possible de l'amorce 12S-V05_R (SEQ ID NO.2) est représentée à la SEQ ID NO. 5. Certaines variantes possibles de l'amorce 12S-V05_F (SEQ ID NO. 1) sont représentées aux SEQ ID Nos. 6-8. Habituellement, les variations de séquence sont plutôt introduites à l'extrémité 5' des oligonucléotides pour ne pas compromettre la réaction d'amplification. Classiquement, on peut par exemple introduire des nucléotides supplémentaires à l'extrémité 5' des oligonucléotides.

Par « séquence capable de s'hybrider de manière sélective » ou « oligonucléotide capable de s'hybrider de manière sélective », on entend selon l'invention les séquences qui s'hybrident avec la séquence de référence à un niveau supérieur au bruit de fond de manière significative. Le niveau du signal généré par l'interaction entre la séquence capable de s'hybrider de manière sélective et les séquences de référence est généralement 10 fois, de préférence 100 fois plus intense que celui de l'interaction des autres séquences d'ADN générant le bruit de fond. Les conditions d'hybridation stringentes permettant une hybridation sélective sont connues de l'homme du métier. En général, la température d'hybridation et de lavage est inférieure d'au moins 5°C, par exemple 10°C, au Tm de la séquence de référence à un pH donné et pour une force ionique donnée. Typiquement, la température d'hybridation est d'au moins 30°C pour un polynucléotide de 15 à 50 nucléotides et d'au moins 60°C pour un polynucléotide de plus de 50 nucléotides. A titre d'exemple, l'hybridation est réalisée dans le tampon suivant : 6X SSC, 50 mM Tris-HCl (pH 7.5), 1 mM EDTA, 0.02% PVP, 0.02% Ficoll, 0.02% BSA, 500 µg/ml sperme de saumon denaturé DNA. Les lavages sont par exemple réalisés successivement à faible stringence dans un tampon 2X SSC, 0,1% SDS, à moyenne stringence dans un tampon 0,5X SSC, 01% SDS et à forte stringence dans un tampon 0,1X SSC, 0,1%SDS. L'hybridation peut bien entendu être effectuée selon d'autres méthodes usuelles connues de l'homme du métier (voir notamment Sambrook, Fristsch et Maniatis, dans leur ouvrage intitulé "Molecular cloning: a laboratory manual", édition : Cold Spring Harbor Laboratory Press, Cold Spring Harbor, NY, 1989).

Par « stringence », on entend la rigueur des conditions opératoires (notamment la température et la force ionique) dans lesquelles se déroule une. hybridation moléculaire.

Le paramètre déterminant dans la spécificité et la réversibilité de l'hybridation moléculaire est le Tm ou température de fusion (melting temperature). C'est la température à laquelle la moitié de l'ADN est sous forme monobrin et l'autre moitié sous forme double brin. Le Tm dépend de nombreux facteurs tels que la longueur du fragment d'ADN considéré, sa richesse en cytosines et guanines et la concentration en sels, notamment en ion Na du milieu réactionnel. En pratique, l'expérimentateur peut créer ou supprimer l'hybridation moléculaire en choisissant une température du milieu réactionnel inférieure, égale ou supérieure au Tm. Les conditions de forte stringence sont celles pour lesquelles la température d'hybridation et de lavage est inférieure d'au moins 5°C et jusqu'à moins 10°C au Tm de la séquence de référence à un pH donné et pour une force ionique donnée.

Par « amplification », on entend toute amplification enzymatique *in vitro* d'une séquence définie d'ADN, notamment du type réaction de polymérisation en chaîne (ou Polymerase Chain Reaction, PCR).

Les amorces selon l'invention peuvent en outre comporter des étiquettes qui vont permettre d'identifier rapidement l'appartenance de chaque séquence amplifiée à un échantillon de départ. Le terme « queue » peut être utilisée de manière équivalente, à la place du terme « étiquette » (« tags » en anglais). Les étiquettes sont de courtes séquences nucléotidiques, généralement de longueur inférieure à 10 nucléotides. Ces étiquettes sont particulièrement utiles lorsqu'il s'agit d'utiliser des séquenceurs de nouvelle génération qui permettent de séquencer près de 100 000 séquences à la fois. En effet, chaque étiquette correspondant à un échantillon de départ à analyser, on peut ainsi mélanger plusieurs produits d'amplification avant de procéder au séquençage. Ceci est avantageux car mettre en oeuvre un séquençage via un séquenceur de nouvelle génération peut s'avérer être une opération coûteuse. Dans la pratique, les amorces selon l'invention peuvent comporter un fragment nucléotidique additionnel du coté 5' spécifique de chaque échantillon. Après amplification de plusieurs échantillons, mélange des différents produits d'amplification ainsi obtenus, et séquençage, chaque séquence amplifiée est ainsi facilement assignée à un échantillon de départ. Les étiquettes constituent donc un outil particulièrement avantageux lorsqu'il s'agit de séquencer à haut débit (Binladen *et al*., 2007).

Habituellement, l'amplification comporte des cycles successifs (généralement de 20 à 40) d'amplification, eux-mêmes composés de trois phases: après une étape de dénaturation (séparation des deux brins de la double hélice) de l'ADN, le positionnement des amorces (courtes séquences d'oligonucléotides spécifiquement choisies) en face de leurs séquences complémentaires, sur les brins d'ADN, et leur fixation sur ces cibles constitue la deuxième phase du procédé (hybridation). La phase d'extension fait intervenir une enzyme, l'ADN polymérase, qui synthétise, à partir des amorces, le brin complémentaire de celui ayant servi de matrice. La répétition de ce cycle conduit à l'amplification exponentielle du fragment d'ADN, également appelé produit d'amplification ou ADN amplifié. Selon un aspect de l'invention, l'hybridation s'effectue à une température comprise entre 45 et 65°C. Notamment, l'hybridation peut s'effectuer à une température comprise entre 45 et 60°C lorsque les amorces ne comportent pas d'étiquette. Alternativement, l'hybridation peut s'effectuer à une température comprise entre 50 et 65°C lorsque les amorces comportent une étiquette.

Selon un autre aspect, l'invention concerne une paire d'oligonucléotides, selon laquelle le premier oligonucélotide s'hybride de manière sélective à la séquence SEQ ID NO. 3 et le deuxième nucléotide s'hybride de manière sélective à la séquence SEQ ID NO. 4 dans des conditions de stringences suffisantes pour l'amplification d'une région variable du gène mitochondrial 12S de l'Homme dont la séquence est représentée à la SEQ ID NO.66 et qui peut être utilisée comme séquence de référence.

Selon un aspect de l'invention, la paire d'oligonucléotides permet l'amplification d'une région du gène mitochondrial 12S qui comporte moins de 120 nucléotides, en particulier moins de 110 ou moins de 100 nucléotides, amorces non comprises (c'est-à-dire sans compter la longueur des amorces). Disposer d'une paire d'amorce universelle qui permette l'amplification d'une région variable courte est très avantageuse car l'utilisation de telles amorces dans des substrats complexes ou dégradés permet la détection et si besoin l'identification des espèces dans de tels substrats.
Les amorces de la présente invention permettent de détecter et identifier plus de 7700 espèces dans le sous-embranchement du règne animal. Parmi ces espèces figurent notamment les amphibiens, les oiseaux, les poissons chondrichtyens, les mammifères, les reptiles et les poissons téléostéens. Les tableaux 1 à 6 donnés dans la partie expérimentale représentent une partie des séquences susceptibles d'être amplifié avec les amorces de la présente invention. Selon un aspect de la présente invention, la région amplifiée par réaction de polymérisation en chaîne présente une séquence nucléotidique sélectionnée dans le groupe consistant en l'une quelconque des SEQ ID NOS. 9-100.

La présente invention concerne également un mélange d'amorces pour l'amplification d'une région variable du gène mitochondrial 12S des vertébrés par réaction de polymérisation en chaîne (PCR) comprenant les amorces d'amplification 12S-V05_F, 12S-V05_R et l'amorce de blocage selon SEQ ID 101. Alternativement, l'invention concerne également un mélange d'amorces pour l'amplification d'une région variable du gène mitochondrial 12S des vertébrés par réaction de polymérisation en chaîne (PCR) comprenant les amorces d'amplification 12S-V05_F et 12S-V05_R et au moins une autre paire d'oligonucléotides choisies parmi les paires d'amorces :
- amorces d'amplification selon SEQ ID NO.102 et SEQ ID NO.103;
- amorces d'amplification selon SEQ ID NO.104 et SEQ ID NO.105;
- amorces d'amplification selon SEQ ID NO.106 et SEQ ID NO.107, éventuellement avec amorce de blocage selon SEQ ID NO.108 ;
- amorces d'amplification selon SEQ ID NO.109 et SEQ ID NO.110;
- amorces d'amplification selon SEQ ID NO.111 et SEQ ID NO.112;
- amorces d'amplification selon SEQ ID NO.113 et SEQ ID NO.114;
- amorces d'amplification selon SEQ ID NO.115 et SEQ ID NO.116;
- amorces d'amplification selon SEQ ID NO.117 et SEQ ID NO.118;
- amorces d'amplification selon SEQ ID NO.119 et SEQ ID NO.120;
- amorces d'amplification selon SEQ ID NO.121 et SEQ ID NO.122, éventuellement avec amorce de blocage selon SEQ ID NO.123 ;
- amorces d'amplification selon SEQ ID NO.124 et SEQ ID NO.125, éventuellement avec amorce de blocage selon SEQ ID NO.126 ;
- amorces d'amplification selon SEQ ID NO.127 et SEQ ID NO.128, éventuellement avec amorce de blocage selon SEQ ID NO.129 ;
- amorces d'amplification selon SEQ ID NO.130 et SEQ ID NO.131, éventuellement avec amorce de blocage selon SEQ ID NO.132 ;
- amorces d'amplification selon SEQ ID NO.133 et SEQ ID NO.134, éventuellement avec amorce de blocage selon SEQ ID NO.135 et/ou SEQ ID NO.136; et
- amorces d'amplification selon SEQ ID NO.137 et SEQ ID NO.138, éventuellement avec amorce de blocage selon SEQ ID NO.139.

### Procédés mettant en oeuvre la paire d'oligonucléotides

La présente invention concerne également un procédé d'amplification d'une région du gène mitochondrial 12S des vertébrés, comprenant les étapes suivantes :
a) on dispose d'un échantillon contenant de l'ADN de vertébrés ;
b) on réalise une réaction d'amplification en chaîne en utilisant une paire d'oligonucléotides selon l'invention.

L'échantillon à partir duquel la réaction d'amplification est réalisée peut être collecté dans l'environnement. Dans ce cas, il peut par exemple s'agir d'un échantillon de sol, eau ou fèces. L'échantillon peut également provenir de l'industrie, par exemple des farines animales ou des plats industriels (aliments transformés) de l'industrie alimentaire. L'échantillon en question peut par exemple provenir d'un produit qui a été congelé, lyophilisé ou chauffé. Un avantage du procédé selon l'invention utilisant les amorces ci-dessus décrites est que l'ADN contenu dans l'échantillon peut se trouver sous forme dégradée. Cela est principalement dû au fait que les amorces ont été choisies de manière à ce que la région amplifiée soit courte, par exemple de longueur inférieure à 120 paires de bases.

Selon un aspect de l'invention, l'hybridation s'effectue à une température comprise entre 45 et 65°C. En fonction du fait que les paires d'oligonucléotides de l'invention comporte ou non une étiquette tel que cela est décrit précédemment, les conditions de l'amplification, en particulier la température d'hybridation, peut ou non varier. Notamment, l'hybridation peut s'effectuer à une température comprise entre 45 et 60°C lorsque les amorces ne comportent pas d'étiquette. Par exemple, on peut utiliser une température de 51-53°C. Alternativement, l'hybridation peut s'effectuer à une température comprise entre 50 et 65°C lorsque les amorces comportent une étiquette. Par exemple, on peut utiliser une température de 57-58°C. L'homme du métier sait adapter la température d'hybridation aux amorces utilisées.

Le procédé d'amplification selon l'invention peut nécessiter une étape d'extraction de l'ADN avant d'effectuer l'amplification. On utilise alors par exemple pour cela un kit d'extraction disponible dans le commerce.

La présente invention propose également un procédé de détection d'une espèce de vertébrés dans un échantillon. Ce procédé de détection selon l'invention comprend les étapes suivantes :
a) on dispose d'un échantillon susceptible de contenir de l'ADN d'espèces vertébrés ;
b) on isole l'ADN total contenu dans l'échantillon ;
c) on réalise une réaction d'amplification en chaîne en utilisant une paire d'oligonucléotides selon l'invention ; et
d) on détecte l'éventuelle présence d'un produit d'amplification.

L'invention se rapporte aussi à un procédé de détection et d'identification d'une espèce de vertébré dans un échantillon, comprenant les étapes suivantes :
a) on dispose d'un échantillon susceptible de contenir de l'ADN d'espèces vertébrés ;
b) on isole l'ADN total contenu dans l'échantillon ;
c) on réalise une réaction d'amplification en chaîne en utilisant une paire d'oligonucléotides selon l'invention ;
d) on détecte la présence d'un produit d'amplification ; et
e) on détermine la séquence du produit d'amplification pour identifier l'espèce de vertébré contenue dans l'échantillon.
L'étape de détermination de la séquence correspond au séquençage de celle-ci. Les méthodes et outils de séquençage sont connus de l'homme du métier et un exemple de ce qui peut être utilisé dans la présente invention est donné dans la partie expérimentale.

### Kit de détection

L'invention a également trait à un kit pour la détection d'une espèce de vertébré dans un échantillon, ledit kit comprenant une paire d'oligonucléotides selon l'invention.

Selon un aspect de l'invention, le kit comprend en outre une amorce de blocage selon l'une quelconque des séquences SEQ ID NOS.101, 108, 123, 126, 129, 132, 135, 136, 139-142. Utiliser une amorce de blocage est utile lorsqu'il s'agit d'éviter l'amplification d'une espèce déterminée, par exemple l'homme ou la vache. Ces amorces sont choisies à partir de l'extrémité la plus variable du fragment amplifié, et de manière à ce que l'amorce de blocage se chevauche avec l'amorce d'amplification sur au moins 6 nucléotides, par exemple sur 8 nucléotides. Enfin, il est nécessaire que le Tm de l'amorce de bloquage soit nettement supérieur à celui des amorces d'amplification (au moins 5°C, par exemple 10°C).

Selon un autre aspect encore de l'invention, le kit comprend au moins une autre paire d'oligonucléotides. Cette au moins une autre paire d'oligonucléotides peut être alors utilisée à titre de vérification du contenu de l'échantillon ou à titre d'alternatives, par exemple si la paire d'oligonucléotides 12S-V05_F (SEQ ID NO. 1) et 12S-V05_R (SEQ ID NO. 2) ne permettait pas l'amplification.

Selon un autre aspect encore de l'invention, le kit comprend en outre une étiquette de moins de 10 nucléotides pour l'identification de l'échantillon de départ après séquençage du produit PCR.

Le kit selon l'invention peut également en outre contenir de manière non limitative des réactifs additionnels, par exemple une enzyme DNA polymerase, des dNTP, du Tris-HCl, du KCI, du MgCl₂ ou du Bovine Serum Albumin (BSA).

### Exemple 1

### Matériels et méthodes

### PCR

Les amorces 12S-V05 ont été testées pour l'analyse du régime alimentaire de trois espèces de félidés :
i) le léopard commun, *Panthera pardus* : un échantillon;
ii) le léopard des neiges, *Uncia uncia* : un échantillon;
iii) le chat-léopard, *Prionailurus bengalensis* : deux échantillons,
en utilisant des fèces comme source d'ADN.

Les extractions d'ADN ont été effectuées à partir de 15 mg de matière, en utilisant un kit d'extraction classique et en suivant les instructions du fabricant (DNeasy Blood and Tissue Kit, QIAgen GmbH, Hilden, Germany).

Les extraits d'ADN ont été récupérés dans un volume de 250 µL.

Les amplifications d'ADN ont été faites dans un volume de 25 µL, en utilisant 2 µL d'extrait par réaction.

Le mélange d'amplification contenait 1 U d'AmpliTaq ® Gold DNA Polymerase (Applied Biosystems, Foster City, CA), 10 mM Tris-HCl, 50 mM KCI, 2 mM MgCl₂, 0,2 mM de chaque dNTP, 0,1 µM de chaque amorce (12SV05F/R + une étiquette ou fragment additionnel de 9 nucléotides du côté 5' spécifique de chaque échantillons), et 5 µg de "bovine serum albumin" (BSA, Roche Diagnostic, Basel, Switzerland).

Le mélange PCR a été dénaturé pendant 10 min à 95°C, suivi de 45 cycles de 30 s à 95°C (dénaturation) et 30 s à 60°C (hybridation).

Comme les séquences cibles sont plus courtes que 120 paires de bases, l'étape d'élongation a été supprimée de manière à réduire la production de l'artefact +A (Brownstein *et al.,* 1996; Magnuson *et al*., 1996) qui réduit l'efficacité de la première étape de séquençage (ligation à bouts francs).

### Séquençage

Le séquençage a été effectué sur un séquenceur Illumina/Solexa Genome Analyzer IIx (Illumina Inc., San Diego, CA 92121 USA), en utilisant le Paired-End Cluster Generation Kit V4 et le Sequencing Kit V4 (Illumina Inc., San Diego, CA 92121 USA), en suivant les instructions du fabriquant.

108 nucléotides ont été séquencés à chaque extrémité des fragments d'ADN.

Les lectures ont été analysées avec les OBITools (http://www.grenoble .prabi.fr/trac/OBITools):
(i) les deux lectures (dans les deux sens) d'un même fragment ont été alignées et fusionnées avec le programme solexaPairEnd, prenant en compte les données de qualité;
(ii) les amorces et les fragments additionnels pour identifier les échantillons ont été identifiés avec le programme ngsfilter;
(iii) seules les régions amplifiées, sans les amorces, ont été conservées pour les analyses ultérieures;
(iv) les séquences strictement identiques ont été regroupées avec le programme obiuniq;
(v) les séquences plus courtes que 10 paires de bases, ou contenant des ambiguïtés, ou présentes moins de 10 fois ont été retirées avec le programme obigrep;
(vi) l'assignation à un taxon a été réalisée avec le programme ecoTag (Pégard *et al.,* 2009); ce programme est basé sur FASTA35 (Pearson et Lipman, 1988) pour trouver des séquences hautement similaires dans une base de référence qui a été construite à partir des données EMBL en utilisant le programme ecoPCR (Ficetola *et al*., 2010).

### Résultats

Lors de cette expérimentation, les séquences suivantes ont pu être identifiées:
Léopard commun (*Panthera pardus*) : numéro d'accession EF551002
Léopard des neiges (*Uncia uncia*)
Chat-léopard (*Prionailurus bengalensis*)
Chèvre (*Capra hircus*) : numéro d'accession AF533441 (SEQ ID NO.56)
Bouquetin de Sibérie (*Capra sibirica*)
Musaraigne (*Crocidura pullata*)
Perdrix chukar (*Alectoris chukar*)
Grenouille (*Nanorana vicina*)

Le tableau ci-dessous présente les nombre de chaque séquence obtenue pour les quatre échantillons.

| | | fèces | | | |
|---|---|---|---|---|---|
| | | Léopard commun | Léopard des neiges | Chat-léopard | |
| | | | | 1 | 2 |
| prédateur | Léopard commun (*Panthera pardus*) | 2460 | - | | |
| | Léopard des neiges (*Uncia uncia*) | - | 10807 | | |
| | Chat-léopard (*Prionailurus bengalensis*) | - | - | 1982 | 9765 |
| proie | Chèvre (*Capra hircus*) | 2969 | | | |
| | Bouquetin de Sibérie (*Capra sibirica*) | | 1256 | | |
| | Musaraigne (*Crocidura pullata*) | | | | 964 |
| | Perdrix chukar (*Alectoris chukar*) | | | 1711 | |
| | Grenouille (*Nanorana vicina*) | | | | 910 |

### PCR réalisée dans les mêmes conditions avec en outre des amorces de blocage

Les mêmes échantillons sont soumis au même protocole pour l'amplification, mais avec l'amorce de blocage à une concentration de 2 µM; toutes les autres concentrations restent identiques.

Les amorces de blocage suivantes ont été utilisées :
Léopard commun :
   SEQ ID NO.140
Léopard des neiges :
   SEQ ID NO.141
Chat-léopard :
   SEQ ID NO.142

Le tableau ci-dessous présente les nombre de chaque séquence obtenue pour les quatre échantillons, mais en utilisant les amorces de blocage pour l'amplification.

| | | fèces | | | |
|---|---|---|---|---|---|
| | | Léopard commun | Léopard des neiges | Chat-léopard | |
| | | | | 1 | 2 |
| prédateur | Léopard commun (*Panthera pardus*) | 10 | - | | |
| | Léopard des neiges (*Uncia uncia*) | - | 318 | | |
| | Chat-léopard (*Prionailurus bengalensis*) | - | - | 0 | 0 |
| proie | Chèvre (*Capra hircus*) | 5951 | | | |
| | Bouquetin de Sibérie (*Capra* | | 6225 | | |
| | *sibirica*) | | | | |
| | Musaraigne (*Crocidura pullata*) | | | 1522 | |
| | Perdrix chukar (*Alectoris chukar*) | | 741 | | |
| | Grenouille (*Nanorana vicina*) | | | 1141 | |
| | Rat noir (*Rattus rattus*) | | | 784 | |

Il est à noter que les amorces étant universelles, les proportions obtenues après séquençage reflètent relativement bien les proportions initiales dans les extraits. Il s'agit d'un autre avantage des amorces utilisées.

### Discussion

Ces résultats démontrent clairement que le système fonctionne, même pour l'analyse d'ADN très dégradé comme celui issu de fèces de carnivores. Dans cet exemple, des séquences de mammifères, d'oiseaux, et de batraciens ont été amplifiées. L'utilisation d'amorces de blocage permet de limiter l'amplification des séquences du prédateur dans le cas présenté ci-dessus, et permet également de bloquer l'amplification de contaminants potentiels.

### Variabilité de la séquence amplifiée

Les tableaux 1 à 6 ci-dessous montrent les variations de la zone amplifiée par les amorces 12S-V05_F et 12S-V05_R de la présente invention pour la détection et l'identification d'espèces de vertébrés. Ces tableaux ont été élaborés à partir de la version 103 de la base de données EMBL.

Il est à noter que ces tableaux ne représentent qu'une partie des espèces pouvant être identifiées en utilisant les amorces de la présente invention. En effet, 24759 séquences sont susceptibles d'être amplifiées en utilisant ces amorces (amplification électronique avec le logiciel ecoPCR, Filetola *et al*., 2010), en tolérant trois mismatches (mais aucun mismatch sur le deux derniers nucléotides du côté 3' des amorces). Ces 24759 séquences sont toutes relatives à des vertébrés dans la mesure où les amorces sont très spécifiques des vertébrés. Plus précisément ces 24759 séquences correspondent à 7726 espèces différentes et à 8264 séquences uniques (deux espèces proches peuvent avoir la même séquence, et une même espèce peut avoir plusieurs variants).

Les tableaux ci-dessous mettent en évidence le fait que deux régions très conservées encadrent une partie variable d'une longueur d'environ 100 nucléotides (voir listage de séquences). Une telle région représente donc une très bonne cible en vue de la détection et de l'identification des espèces de vertébrés à partir d'extraits d'ADN complexes et dégradés.

### Exemple 2

### Comparaison des amorces de la présente invention (12S-V05_F selon SEQ ID NO.1 et 12S-V05_R selon SEQ ID NO.2) avec les amorces décrites dans WO 2006/024751

La comparaison a été effectuée sur l'ensemble des ADN mitochondriaux d'animaux téléchargés le 15 Mars 2011 à partir d'EMBL. Cette base contient un total de 2424 séquences, dont 1496 vertébrés et 928 non-vertébrés.

Les amplifications ont été simulées à l'aide du logiciel ecoPCR (Ficetola *et al.* 2010; Bellemain *et al.* 2010), en tolérant un maximum de trois mésappariement par amorce, mais en fixant les deux derniers nucléotides du côté 3' (pas de mésappariement possible sur les deux derniers nucléotides du côté 3').

Les amorces suivantes ont été testées:
(i) L15162/H15392 (amplifiant un fragment de 129 nucléotides)
(ii) L15411/H15497 (amplifiant un fragment de 85 nucléotides)
(iii) 12S_V5_F/12_V5_R (amplifiant un fragment d'environ 100 nucléotides)

Les résultats obtenus sont les suivants (nombre d'amplification positives):

| Amorces utilisées | Séquences de vertébrés amplifiées | | Séquences de non-vertébrés amplifiées | |
|---|---|---|---|---|
| | nombre | % | nombre | % |
| L15162/H15392 | 1488 | 99,47 | 396 | 42,67 |
| L15411/H15497 | 1354 | 90,51 | 331 | 35,67 |
| 12S-V05_F/12-V05_R | 1388 | 92,78 | 0 | 0 |

Nous avons observé que les amorces L15162/H15392 amplifient deux séquences cibles pour les ADN mitochondriaux avec les numéros d'accession NC_010292 et NC_008290. De même les amorces L15411/H15497 amplifient sept séquences cibles pour NC_006331, et deux séquences cibles pour NC_015087, NC_015085, NC_014681, NC_014679, NC_014280, NC_011598, NC_011597, NC-008360, NC_008290, NC_007782 et NC_006992.

Les amorces de la présente invention n'amplifient jamais deux séquences cibles sur le même ADN mitochondrial.

D'autre part, en testant les amorces sur la version 107 d'EMBL comprenant l'ensemble des séquences déposées dans les bases de données publiques, nous avons constaté que L15162/H15392 et L15411/H15497 amplifient également quelques bactéries, et quelques végétaux, ce qui n'est jamais le cas pour les amorces la présente invention. Les émorces de la présente invention sont beaucoup plus spécifiques.

L'extrême dégénérescence des amorces L15162/H15392 et L15411/H15497 explique qu'elles amplifient une bonne proportion de l'ensemble des vertébrés. En revanche, cette dégénérescence explique aussi les artéfacts produits (plusieurs séquences cibles amplifiées sur un même ADN mitochondrial) et le manque de spécificité pour les vertébrés (L15162/H15392 amplifient plus de 40% de non-vertébrés, et L15411/H15497 plus de 35%).

**Tableau 1 - Amphibiens**

| Nom scientifique | Numéro d'accession | Séquence correspondant à 12S-V05_F | Séquence amplifiée | Séquence correspondant à 12S-V05_F |
|---|---|---|---|---|
| *Alytes obstetricans* | AJ440759 | TTAGATACCCCACTATGC | | TAGAACAGGCTCCTCTAG |
| *Bombina variegata* | AJ440764 | TTAGATACCCCACTATGC | | TAGAACAGGCTCCTCTAG |
| *Bufo bufo* | AY325988 | TTAGATACCCCACTATGC | | TAGGACAGGCTCCTCTAG |
| *Bufo viridis* | AY680267 | TTAGATACCCCACTATGC | | TAGGACAGGCTCCTCTAG |
| *Dendrobates tinctorius* | DQ502123 | TTAGATACCCCACTATGC | | TAGGACAGGCTCCTCTAG |
| *Hyla arborea* | AY843601 | TTAGATACCCCACTATGC | | CAGGACAGGCTCCTCTAG |
| *Hyla meridionalis* | AY819370 | TTAGATACCCCACTATGC | | TAGGACAGGCTCCTCTAG |
| *Rana pipiens* | DQ283123 | TTAGATACCCCACTATGC | | AAGAACAGGCTCCTCTAG |
| *Rana temporaria* | AY326063 | TTAGATACCCCACTATGC | | TAGAACAGGCTCCTCTAG |
| *Salamandra atra* | AY928616 | TTAGATACCCCACTATGC | | TAGAACAGGCTCCTCTAG |
| *Salamandra lanzai* | AY928617 | TTAGATACCCCACTATGC | | TAGAACAGGCTCCTCTAG |

**Tableau 2 - Oiseaux**

| Nom scientifique | Numéro d'accession | Séquence correspondant à 12S-V05_F | Séquence amplifiée | Séquence correspondant à 12S-V05_F |
|---|---|---|---|---|
| *Accipiter gentilis* | AB219551 | TTAGATACCCCACTATGC | | TAGAACAGGCTCCTCTAG |
| *Acrocephalus arundinaceus* | AB492871 | TTAGATACCCCACTATGC | | CAGAACAGGCTCCTCTAG |
| *Alectoris rufa* | AM902517 | TTAGATACCCCACTATGC | | TAGAACAGGCTCCTCTAG |
| *Anser anser* | AY164530 | TTAGATACCCCACTATGC | | TAGAACAGGCTCCTCTAG |
| *Aquila chrysaetos* | AB219549 | TTAGATACCCCACTATGC | | CAGAACAGGCTCCTCTAG |
| *Ardea cinerea* | AF407136 | TTAGATACCCCACTATGC | | TAGAACAGGCTCCTCTAG |
| *Asio flammeus* | AF406881 | TTAGATACCCCACTATGC | | TAGGACAGGCTCCTCTAG |
| *Asio otus* | AY274022 | TTAGATACCCCACTATGC | | CAGGACAGGCTCCTCTAG |
| *Bubo bubo* | AY513589 | TTAGATACCCCACTATGC | | TAGGACAGGCTCCTCTAG |
| *Buteo buteo* | AF380305 | TTAGATACCCCACTATGC | | TAGAACAGGCTCCTCTAG |
| *Cardinalis cardinalis* | AF447215 | TTAGATACCCCACTATGC | | CAGAACAGGCTCCTCTAG |
| *Ciconia ciconia* | AB026818 | TTAGATACCCCACTATGC | | CAGAACAGGCTCCTCTAG |
| *Columbalivia* | AF173585 | TTAGATACCCCACTATGC | | TAGAACAGGCTCCTCTAG |
| *Corvus corone* | AF386463 | TTAGATACCCCACTATGC | | CAGAACAGGCTCCTCTAG |
| *Cygnus cygnus* | AY164523 | TTAGATACCCCACTATGC | | TAGAACAGGCTCCTCTAG |
| *Falco peregrinus* | AB219556 | TTAGATACCCCACTATGC | | CAGAACAGGCTCCTCTAG |
| *Fringilla coelebs* | AF407088 | TTAGATACCCCACTATGC | | CAGAACAGGCTCCTCTAG |
| *Gallus gallus* | AB086102 | TTAGATACCCCACTATGC | | TAGAACAGGCTCCTCTAG |
| *Lagopus muta* | AF222585 | TTAGATACCCCACTATGC | | TAGAACAGGCTCCTCTAG |
| *Motacilla alba* | AF407083 | TTAGATACCCCACTATGC | | CAGAACAGGCTCCTCTAG |
| *Parus major* | AY136557 | TTAGATACCCCACTATGC | | TAGAACAGGCTCCTCTAG |
| *Passeur domesticus* | AF407085 | TTAGATACCCCACTATGC | | CAGAACAGGCTCCTCTAG |
| *Perdix perdix* | AF222590 | TTAGATACCCCACTATGC | | CAGAACAGGCTCCTCTAG |
| *Phasianus colchicus* | AM902515 | TTAGATACCCCACTATGC | | TAGAACAGGCTCCTCTAG |
| *Sylvia atricapilla* | AM889140 | TTAGATACCCCACTATGC | | CAGAACAGGCTCCTCTAG |
| *Tetrao urogallus* | AF222594 | TTAGATACCCCACTATGC | | TAGAACAGGCTCCTCTAG |
| *Turdus philomelos* | AF484935 | TTAGATACCCCACTATGC | | CAGAACAGGCTCCTNTAG |
| *Zosterops pallidus* | AY136570 | TTAGATACCCCACTATGC | | CAGAACAGGCTCCTCTAG |

**Tableau 3 - Poissons chondrichtyens**

| Nom scientifique | Numéro d'accession | Séquence correspondant à 12S-V05_F | Séquence amplifiée | Séquence correspondant à 12S-V05_F |
|---|---|---|---|---|
| *Chimaera monstrosa* | AJ310140 | TTAGATACCCCACTATGT | | TAGAACAGGCTCCTCTAG |
| *Manta birostris* | AF448000 | TTAGATACCCTACTATGC | | TAGAACAGGCTCCTCTAG |
| *Raja brachyura* | EF081263 | TTAGATACCCTACTATGC | | TAGAACAGGCTCCTCTAG |
| *Squatina nebulosa* | AF448031 | TTAGATACCCTACTATGC | | CAGAACAGGCTCCTCTAG |
| *Torpedo tokionis* | AF448026 | TTAGATACCCTACTATGT | | CAGAACAGGCTCCTCTAG |

**Tableau 4 - Mammifères**

| Nom scientifique | Numéro d'accession | Séquence correspondant à 12S-V05_F | Séquence amplifiée | Séquence correspondant à 12S-V05_F |
|---|---|---|---|---|
| *Bison bison* | AF091687 | TTAGATACCCCACTATGC | | TAGAACAGGCTCCTCTAG |
| *Bos taurus* | AB074962 | TTAGATACCCCACTATGC | | TAGAACAGGCTCCTCTAG |
| *Canis lupus* | AB048585 | TTAGATACCCCACTATGC | | TAGAACAGGCTCCTCTAG |
| *Capra hircus* | AF533441 | TTAGATACCCCACTATGC | | TAGAACAGGCTCCTCTAG |
| *Capra ibex* | AY846815 | TTAGATACCCCACTATGC | | TAGAACAGGCTCCTCTAG |
| *Capreolus capreolus* | AJ885202 | TTAGATACCCCACTATGC | | TAGAACAGGCTCCTCTAG |
| *Castor fiber* | AJ389542 | TTAGATACCCCACTATGC | | TAGAACAGGCTCCTCTAG |
| *Cervus elaphus* | AB245427 | TTAGATACCCCACTATGC | | TAGAACAGGCTCCTCTAG |
| *Eliomys quercinus* | Y16896 | TTAGATACCCCACTATGC | | TAGAACAGGCTCCTCTAG |
| *Eptesicus serotinus* | AY495467 | TTAGATACCCCACTATGC | | TAGAACAGGCTCCTCTAG |
| *Erinaceus europaeus* | X88898 | TTAGATACCCTATTATGC | | CAGAACAGGCTCCTCTAG |
| *Felis catus* | AY012149 | TTAGATACCCCACTATGC | | TAGAACAGGCTCCTCTAG |
| *Glis glis* | AJ001562 | TTAGATACCCCACTATGC | | TAGAACAGGCTCCTCTAG |
| *Homo sapiens* | AB055387 | TTAGATACCCCACTATGC | | CAGAACAGGCTCCTCTAG |
| *Lepus europaeus* | AJ421471 | TTAGATACCCCATTATGC | | TAGAACAGGCTCCTCTAG |
| *Lutra lutra* | FJ236015 | TTAGATACCCCACTATGC | | TAGAACAGGCTCCTCTAG |
| *Meles meles* | AB119077 | TTAGATACCCCACTATGC | | TAGAACAGGCTCCTCTAG |
| *Mus musculus* | AB042432 | TTAGATACCCCACTATGC | | TAGAACAGGCTCCTCTAG |
| *Mustela erminea* | AB119066 | TTAGATACCCCACTATGC | | TAGAACAGGCTCCTCTAG |
| *Myodes glareolus* | AJ005781 | TTAGATACCCCACTATGC | | TAGAACAGGCTCCTCTAG |
| *Oryctolagus cuniculus* | AJ001588 | TTAGATACCCCACTATGC | | CGGAACAGGCTCCTCTAG |
| *Ovis aries* | AF010406 | TTAGATACCCCACTATGC | | TAGAACAGGCTCCTCTAG |
| *Rattus norvegicus* | AB 183258 | TTAGATACCCCACTATGC | | TAGAACAGGCTCCTCTAG |
| *Sciurus vulgaris* | AJ238588 | TTAGATACCCCACTATGC | | TAGAACAGGCTCCTCTAG |
| *Sorex araneus* | AY012102 | TTAGATACCCCACTATGC | | CAGAACAGGCTCCTCTAG |
| *Sus scrofa* | AB292606 | TTAGATACCCCACTATGC | | TAGAACAGGCTCCTCTAG |
| *Ursus arctos* | AF303110 | TTAGATACCCCACTATGC | | TAGAACAGGCTCCTCTAG |

**Tableau 5 - Reptiles**

| Nom scientifique | Numéro d'accession | Séquence correspondant à 12S-V05_F | Séquence amplifiée | Séquence correspondant à 12S-V05_F |
|---|---|---|---|---|
| *Coronella austriaca* | AY122836 | TTAGATACCTTACTATGC | | TTAGACAGGCTCCTCTAG |
| *Coronella girondica* | AY122835 | TTAGATACCTTACTATGC | | TTAGACAGGCTCCTCTAG |
| *Gekko gecko* | AY282753 | TTAGATACCCTACTATGC | | TAGGACAGGCTCCTCTAG |
| *Lacerta schreiberi* | AF206591 | TTAGATACCCTATTATGC | | TAGGACAGGCTCCTCTAG |
| *Naja naja* | AF236683 | TTAGATACCTTACTATGC | | TTAGACAGGCTCCTCTAG |
| *Natrix maura* | AF402623 | TTAGATACCTTACTATGC | | TTAGACAGGCTCCTCTAG |
| *Natrix natrix* | AY122682 | TTAGATACCTTACTATGC | | TTAGACAGGCTCCTCTAG |
| *Podarcis muralis* | AF206600 | TTAGATACCCTACTATGC | | TAGGACAGGCTCCTCTAG |
| *Vipera ursinii* | AF236687 | TTAGATACCTTACTATAC | | TTAGACAGGCTCCTCTAG |

**Tableau 6 - Poissons téléostéens**

| Nom scientifique | Numéro d'accession | Séquence correspondant à 12S-V05_F | Séquence amplifiée | Séquence correspondant à 12S-V05_F |
|---|---|---|---|---|
| *A cipenser persicus* | AY544139 | TTAGATACCCCACTATGC | | TAGAACAGGCTCCTCTAG |
| *Anguilla anguilla* | AB021887 | TTAGATACCCCACTATGC | | TAGAACAGGCTCCTCTAG |
| *Barbus barbus* | AB238965 | TTAGATACCCCACTATGC | | TAGAACAGGCTCCTCTAG |
| *Coregonus lavaretus* | AB034824 | TTAGATACCCCACTATGC | | TAGAACAGGCTCCTCTAG |
| *Cottus gobio* | AB188189 | TTAGATACCCCACTATGC | | TAGAACAGGCTCCTCTAG |
| *Gadus morhua* | AM489716 | TTAGATACCCCACTATGT | | TAGAACAGGCTCCTCTAG |
| *Gasterosteus aculeatus* | AP002944 | TTAGATACCCCACTATGC | | TAGAACAGGCTCCTCTAG |
| *Labrus merula* | AJ810141 | TTAGATACCCCACTATGC | | TAGAACAGGCTCCTCTAG |
| *Lota Iota* | AP004412 | TTAGATACCCCACTATGT | | TAGAACAGGCTCCTCTAG |
| *Oncorhynchus mykiss* | DQ288268 | TTAGATACCCCACTATGC | | TAGAACAGGCTCCTCTAG |
| *Salvelinus fontinalis* | AF154850 | TTAGATACCCCACTATGC | | TAGAACAGGCTCCTCTAG |
| *Solea solea* | AF488492 | TTAGATACCCCACTATGC | | TAGAACAGGCTCCTCTAG |

## Revendications

1. Paire d'oligonucléotides, selon laquelle le premier oligonucléotide s'hybride de manière sélective à la séquence SEQ ID NO. 3 et le deuxième oligonucléotide s'hybride de manière sélective à la séquence SEQ ID NO. 4 dans des conditions de stringences suffisantes pour l'amplification d'une région variable du gène mitochondrial 12S des vertébrés par réaction de polymérisation en chaîne (PCR).

2. Paire d'oligonucléotides selon la revendication 1, selon laquelle la région amplifiée du gène mitochondrial 12S comporte moins de 110 nucléotides.

3. Paire d'oligonucléotides selon la revendication 1 ou 2, selon laquelle le premier oligonucléotide présente une séquence nucléotidique sélectionnée dans le groupe consistant en SEQ ID NO. 1 et SEQ ID NO. 6-8 et le deuxième oligonucléotide présente une séquence nucléotidique sélectionnée dans le groupe consistant en SEQ ID NO. 2 et SEQ ID NO. 5.

4. Paire d'oligonucléotides selon l'une quelconque des revendications précédentes, selon laquelle la région amplifiée par réaction de polymérisation en chaîne présente une séquence nucléotidique sélectionnée dans le groupe consistant en SEQ ID NO. 9-100.

5. Procédé d'amplification d'une région du gène mitochondrial 12S, comprenant les étapes suivantes :
a) on dispose d'un échantillon susceptible de contenir de l'ADN d'espèces vertébrés ;
b) on réalise une réaction d'amplification en chaîne en utilisant une paire d'oligonucléotides selon l'une quelconque des revendications 1 à 4.

6. Procédé de détection d'une espèce vertébrée dans un échantillon, comprenant les étapes suivantes :
a) on dispose d'un échantillon susceptible de contenir de l'ADN d'espèces vertébrés ;
b) on isole l'ADN total contenu dans l'échantillon ;
c) on réalise une réaction d'amplification en chaîne en utilisant une paire d'oligonucléotides selon l'une quelconque des revendications 1 à 4 ; et
d) on détecte l'éventuelle présence d'un produit d'amplification.

7. Procédé de détection et d'identification d'une espèce vertébrée dans un échantillon, comprenant les étapes suivantes :
a) on dispose d'un échantillon susceptible de contenir de l'ADN d'espèces vertébrés ;
b) on isole l'ADN total contenu dans l'échantillon ;
c) on réalise une réaction d'amplification en chaîne en utilisant une paire d'oligonucléotides selon l'une quelconque des revendications 1 à 4 ;
d) on détecte la présence d'un produit d'amplification ; et
e) on détermine la séquence du produit d'amplification pour identifier l'espèce de vertébré contenue dans l'échantillon.

8. Kit pour la détection d'une espèce de vertébré dans un échantillon, comprenant une paire d'oligonucléotides selon l'une quelconque des revendications 1 à 4 et au moins un réactif additionnel.

9. Kit selon la revendication 8, comprenant en outre une amorce de blocage selon SEQ ID NO. 101.

10. Kit selon la revendication 8 ou 9, comprenant en outre au moins une autre paire d'oligonucléotides choisis parmi les paires d'amorces :
- amorces d'amplification selon SEQ ID NO.102 et SEQ ID NO.103;
- amorces d'amplification selon SEQ ID NO.104 et SEQ ID NO.105;
- amorces d'amplification selon SEQ ID NO.106 et SEQ ID NO.107, éventuellement avec amorce de blocage selon SEQ ID NO.108;
- amorces d'amplification selon SEQ ID NO.109 et SEQ ID NO.110;
- amorces d'amplification selon SEQ ID NO.111 et SEQ ID NO.112;
- amorces d'amplification selon SEQ ID NO.113 et SEQ ID NO.114;
- amorces d'amplification selon SEQ ID NO.115 et SEQ ID NO.116;
- amorces d'amplification selon SEQ ID NO.117 et SEQ ID NO.118;
- amorces d'amplification selon SEQ ID NO.119 et SEQ ID NO.120;
- amorces d'amplification selon SEQ ID NO.121 et SEQ ID NO.122, éventuellement avec amorce de blocage selon SEQ ID NO.123;
- amorces d'amplification selon SEQ ID NO.124 et SEQ ID NO.125, éventuellement avec amorce de blocage selon SEQ ID NO.126 ;
- amorces d'amplification selon SEQ ID NO.127 et SEQ ID NO.128, éventuellement avec amorce de blocage selon SEQ ID NO.129;
- amorces d'amplification selon SEQ ID NO.130 et SEQ ID NO.131, éventuellement avec amorce de blocage selon SEQ ID NO.132 ;
- amorces d'amplification selon SEQ ID NO.133 et SEQ ID NO.134, éventuellement avec amorce de blocage selon SEQ ID NO.135 et/ou SEQ ID NO.136; et
- amorces d'amplification selon SEQ ID NO.137 et SEQ ID NO.138, éventuellement avec amorce de blocage selon SEQ ID NO.139.

## Patentansprüche

1. Oligonukleotidpaar, wobei das erste Oligonukleotid auf selektive Weise zur Sequenz SEQ ID NO. 3 und das zweite Oligonukleotid auf selektive Weise zur Sequenz SEQ ID NO. 4 unter stringenten Bedingungen hybridisiert, die ausreichend für die Erweiterung einer variablen Region des mitochondrialen Gens 12S der Wirbeltiere durch Polymerasekettenreaktion (PCR) sind.

2. Oligonukleotidpaar nach Anspruch 1, wobei die erweiterte Region des mitochondrialen Gens 12S mindestens 110 Nukleotide umfasst.

3. Oligonukleotidpaar nach Anspruch 1 oder 2, wobei das erste Oligonukleotid eine Nukleotidsequenz aufweist, ausgewählt aus der Gruppe, bestehend aus SEQ ID NO. 1 und SEQ ID NO. 6 - 8, und das zweite Oligonukleotid eine Nukleotidsequenz aufweist, ausgewählt aus der Gruppe, bestehend aus SEQ ID NO. 2 und SEQ ID NO. 5.

4. Oligonukleotidpaar nach einem der vorhergehenden Ansprüche, wobei die Region, erweitert durch Polymerasekettenreaktion, eine Nukleotidsequenz aufweist, ausgewählt aus der Gruppe, bestehend aus SEQ ID NO. 9 - 100.

5. Verfahren zur Erweiterung einer Region des mitochondrialen Gens 12S, umfassend die folgenden Schritte:
a) Verfügen über eine Probe, die DNA von Wirbeltierarten enthalten kann;
b) Durchführen einer Erweiterungskettenreaktion unter Verwendung eines Oligonukleotidpaars nach einem der Ansprüche 1 bis 4.

6. Verfahren zum Nachweis einer Wirbeltierart in einer Probe, umfassend die folgenden Schritte:
a) Verfügen über eine Probe, die DNA von Wirbeltierarten enthalten kann;
b) Isolieren der gesamten DNA, enthalten in der Probe;
c) Durchführen einer Erweiterungskettenreaktion unter Verwendung eines Oligonukleotidpaars nach einem der Ansprüche 1 bis 4; und
d) Nachweisen der eventuellen Anwesenheit eines Erweiterungsprodukts.

7. Verfahren zum Nachweis einer Wirbeltierart in einer Probe, umfassend die folgenden Schritte:
a) Verfügen über eine Probe, die DNA von Wirbeltierarten enthalten kann;
b) Isolieren der gesamten DNA, enthalten in der Probe;
c) Durchführen einer Erweiterungskettenreaktion unter Verwendung eines Oligonukleotidpaars nach einem der Ansprüche 1 bis 4;
d) Nachweisen der Anwesenheit eines Erweiterungsprodukts; und
e) Bestimmen der Sequenz des Erweiterungsprodukts, um die Wirbeltierart zu identifizieren, die in der Probe enthalten ist.

8. Kit zum Nachweis einer Wirbeltierart in einer Probe, umfassend ein Oligonukleotidpaar nach einem der Ansprüche 1 bis 4, und mindestens ein zusätzliches Reagens.

9. Kit nach Anspruch 8, umfassend außerdem einen Blockierungsprimer gemäß SEQ ID NO. 101.

10. Kit nach Anspruch 8 oder 9, umfassend außerdem mindestens ein weiteres Oligonukleotidpaar, ausgewählt aus den folgenden Primerpaaren:
- Erweiterungsprimer gemäß SEQ ID NO. 102 und SEQ ID NO. 103;
- Erweiterungsprimer gemäß SEQ ID NO. 104 und SEQ ID NO. 105;
- Erweiterungsprimer gemäß SEQ ID NO. 106 und SEQ ID NO. 107, eventuell mit Blockierungsprimer gemäß SEQ ID NO. 108;
- Erweiterungsprimer gemäß SEQ ID NO. 109 und SEQ ID NO. 110;
- Erweiterungsprimer gemäß SEQ ID NO. 111 und SEQ ID NO. 112;
- Erweiterungsprimer gemäß SEQ ID NO. 113 und SEQ ID NO. 114;
- Erweiterungsprimer gemäß SEQ ID NO. 115 und SEQ ID NO. 116;
- Erweiterungsprimer gemäß SEQ ID NO. 117 und SEQ ID NO. 118;
- Erweiterungsprimer gemäß SEQ ID NO. 119 und SEQ ID NO. 120;
- Erweiterungsprimer gemäß SEQ ID NO. 121 und SEQ ID NO. 122, eventuell mit Blockierungsprimer gemäß SEQ ID NO. 123;
- Erweiterungsprimer gemäß SEQ ID NO. 124 und SEQ ID NO. 125, eventuell mit Blockierungsprimer gemäß SEQ ID NO. 126;
- Erweiterungsprimer gemäß SEQ ID NO. 127 und SEQ ID NO. 128, eventuell mit Blockierungsprimer gemäß SEQ ID NO. 129;
- Erweiterungsprimer gemäß SEQ ID NO. 130 und SEQ ID NO. 131, eventuell mit Blockierungsprimer gemäß SEQ ID NO. 132;
- Erweiterungsprimer gemäß SEQ ID NO.133 und SEQ ID NO. 134, eventuell mit Blockierungsprimer gemäß SEQ ID NO. 135 und/oder SEQ ID NO. 136; und
- Erweiterungsprimer gemäß SEQ ID NO. 137 und SEQ ID NO. 138, eventuell mit Blockierungsprimer gemäß SEQ ID NO. 139.

## Claims

1. A pair of oligonucleotides, according to which the first oligonucleotide selectively hybridizes to the sequence SEQ ID NO. 3 and the second oligonucleotide selectively hybridizes to the sequence SEQ ID NO. 4 under conditions of sufficient stringencies for the amplification of a variable region of the mitochondrial gene 12S of the vertebrates by polymerase chain reaction (PCR).

2. The pair of oligonucleotides according to claim 1, according to which the amplified region of the mitochondrial gene 12S includes lower than 110 nucleotides.

3. The pair of oligonucleotides according to claim 1 or 2, according to which the first oligonucleotide has a nucleotide sequence selected from the group consisting of SEQ ID NO. 1 and SEQ ID NO. 6-8 and the second oligonucleotide has a nucleotide sequence selected from the group consisting of SEQ ID NO. 2 and SEQ ID NO. 5.

4. The pair of oligonucleotides according to any one of the preceding claims, according to which the amplified region by polymerase chain reaction has a nucleotide sequence selected from the group consisting of SEQ ID NO. 9-100.

5. A method for amplifying a region of the mitochondrial gene 12S, comprising the following steps:
a) having a sample likely to contain DNA of vertebrate species;
b) carrying out a chain amplification reaction using a pair of oligonucleotides according to any one of claims 1 to 4.

6. A method for detecting a vertebrate species in a sample, comprising the following steps:
a) having a sample likely to contain DNA of vertebrate species;
b) isolating the total DNA contained in the sample;
c) carrying out a chain amplification reaction using a pair of oligonucleotides according to any one of claims 1 to 4; and
d) detecting the possible presence of an amplification product.

7. The method for detecting and identifying a vertebrate species in a sample, comprising the following steps:
a) having a sample likely to contain DNA of vertebrate species;
b) isolating the total DNA contained in the sample;
c) carrying out a chain amplification reaction using a pair of oligonucleotides according to any one of claims 1 to 4;
d) detecting the possible presence of an amplification product; and
e) determining the sequence of the amplification product in order to identify the vertebrate species contained in the sample.

8. A kit for detecting a vertebrate species in a sample, comprising a pair of oligonucleotides according to any one of claims 1 to 4 and at least one additional reagent.

9. The kit according to claim 8, further comprising a blocking primer according to SEQ ID NO. 101.

10. The kit according to claim 8 or 9, further comprising at least another pair of oligonucleotides selected among the pairs of primers:
- amplification primers according to SEQ ID NO. 102 and SEQ ID NO. 103;
- amplification primers according to SEQ ID NO. 104 and SEQ ID NO. 105;
- amplification primers according to SEQ ID NO. 106 and SEQ ID NO. 107, possibly with blocking primer according to SEQ ID NO. 108;
- amplification primers according to SEQ ID NO. 109 and SEQ ID NO. 110;
- amplification primers according to SEQ ID NO. 111 and SEQ ID NO. 112;
- amplification primers according to SEQ ID NO. 113 and SEQ ID NO. 114;
- amplification primers according to SEQ ID NO. 115 and SEQ ID NO. 116;
- amplification primers according to SEQ ID NO. 117 and SEQ ID NO. 118;
- amplification primers according to SEQ ID NO. 119 and SEQ ID NO. 120;
- amplification primers according to SEQ ID NO. 121 and SEQ ID NO. 122, possibly with blocking primer according to SEQ ID NO. 123;
- amplification primers according to SEQ ID NO. 124 and SEQ ID NO. 125, possibly with blocking primer according to SEQ ID NO. 126;
- amplification primers according to SEQ ID NO. 127 and SEQ ID NO. 128, possibly with blocking primer according to SEQ ID NO. 129;
- amplification primers according to SEQ ID NO. 130 and SEQ ID NO. 131, possibly with blocking primer according to SEQ ID NO. 132;
- amplification primers according to SEQ ID NO. 133 and SEQ ID NO. 134, possibly with blocking primer according to SEQ ID NO. 135 and/or SEQ ID NO. 136; and
- amplification primers according to SEQ ID NO. 137 and SEQ ID NO. 138, possibly with blocking primer according to SEQ ID NO. 139.
